# EUROPEAN PATENT APPLICATION

(11) **EP 3 939 497 A1**
(43) Date of publication of application: **19.01.2022**
(21) Application number: 20382637.5
(22) Date of filing: 15.07.2020
(51) Int. Cl.: A61B 5/00

(54) **BIDIRECTIONAL MEDICAL DEVICES FOR MONITORING AND STIMULATING NEURONS**

(71) Applicant: Fundación Imdea Nanociencia, 28049 Madrid (ES); Centre National de la Recherche Scientifique (CNRS), 75794 Paris Cedex 16 (FR); Scuola Internazionale Superiore Di Studi Avanzati, 34136 Trieste (IT); Consejo Superior De Investigaciones Científicas, 28006 Madrid (ES); Servicio de Salud de Castilla-la Mancha (SESCAM), 45071 Toledo (ES)
(72) Inventor: GONZÁLEZ PÉREZ, María Teresa, E-28049 Madrid (ES); CAMARERO DE DIEGO, Julio, E-28049 Madrid (ES); PÉREZ GARCÍA, Lucas, E-28049 Madrid (ES); RODRÍGUEZ FERNÁNDEZ, María Isabel, E-28049 Madrid (ES); MIRANDA SORIANO, Rodolfo, E-28049 Madrid (ES); PERNA, Paolo, E-28049 Madrid (ES); MARTÍNEZ RAMÍREZ, Isidoro, E-28049 Madrid (ES); GUERRERO SÁNCHEZ, Rubén, E-28049 Madrid (ES); RODILLA GONZÁLEZ, Beatriz Loreto, E-28049 Madrid (ES); ARCHÉ NÚÑEZ, Ana, E-28049 Madrid (ES); VERA GARCÍA, Arturo, E-28049 Madrid (ES); ENGER, Luiz Guilherme, 14050 Caen, France (FR); FLAMENT, Stéphane, 14050 Caen, France (FR); GUILLET, Bruno, 14050 Caen, France (FR); LAM CHOK SING, Marc Ah-Liong, 14050 Caen, France (FR); MECHIN, Laurence Liliane, 14050 Caen, France (FR); ROUSSEAU, Olivier Henri Marc, 14050 Caen, France (FR); BALLERINI, Laura, 34136 Trieste (IT); SCAINI, Denis, 34136 Trieste (IT); CALARESU, Ivo, 34136 Trieste (IT); SERRANO LÓPEZ-TERRADAS, María Concepción, E - 28049 Madrid (ES); DOMÍNGUEZ BAJO, Ana, E - 28049 Madrid (ES); LÓPEZ DOLADO, Elisa, E - 45071 Toledo (ES); GONZÁLEZ MAYORGA, Francisco Ankor, E - 45071 Toledo (ES); LEBARGY, Sylvain, 14050 Caen (FR)
(74) Representative: ABG Intellectual Property Law, S.L.

(57) **Abstract**

The present invention relates to a probe, a monitoring device, a method for monitoring, the use of the monitoring device, a medical device A, a medical device B, methods for cell monitoring and stimulating, the use of medical device A and the use of medical device B.

## Description

### FIELD OF THE ART

The present invention relates to the field of medical devices for monitoring and stimulation.

### STATE OF THE ART

Electrical stimulation such as neuromuscular stimulation (the electrical excitation of nerves and/or muscle), neuromodulation stimulation (the electrical excitation of nerves, often afferent nerves) and brain stimulation (the stimulation of cerebral or other central nervous system tissue) may provide functional and/or therapeutic outcomes. While existing systems and methods can provide remarkable benefits to individuals requiring neuromuscular or neuromodulation stimulation, many limitations and issues still remain.

Today there are a wide variety of external and implantable medical devices related with electrical stimulation that can be used to provide beneficial results in diverse therapeutic and functional restorations indications. For example, US2018021587A1 describes an implantable pulse generator system and method for functional therapeutic stimulation of muscles and/or nerves and/or central nervous tissue.

However, there is only few medical devices developed that are able to stimulate and monitor a signal at the same time. For example, US2018/0353284A1 describes a neural modulation system comprising magnetic neural stimulators and neural sensors such as tetrodes; wherein the magnetic neural stimulators comprise three dimensional coils. WO2014004557 describes a medical device comprising a magnetic field sensing device such as a reed switch or a Hall sensor device configured for outputting a signal in response to sensing a magnetic field. Most medical devices have limitations regarding signal sensitivity, portability, treatment regimens and usability. In addition, implantable devices have additional limitations related to their reduced size and price.

Thus, there is a need in the art for developing new medical devices that overcome previous limitations.

### BRIEF DESCRIPTION OF THE INVENTION

The object of the present invention is the development of probes and medical devices for monitoring and stimulation of cells.

The probes and medical devices of the present invention are biocompatible, compact, usable and are able to measure ultra-low magnetic signals. In addition, the probes and medical devices of the present invention might be implantable.

Therefore, in a first aspect, the invention is directed to a probe (1.1) comprising at least one sensor or sensor array adapted for monitoring a signal; wherein the probe (1.1) is biocompatible and optionally implantable; wherein the signal monitored by the at least one sensor or sensor array is a magnetic signal.

In a second aspect, the invention is directed to a monitoring device (1) comprising
i. two probes (1.1) as defined in the present invention in any of its particular embodiments; wherein the two probes (1.1) are physically separated a distance of between 0.1 and 100 mm; and
ii. signal processing means (1.4) adapted for processing the signals of the probes;
wherein the two probes (1.1), and the signal processing means (1.4) are operatively connected; and wherein the monitoring device (1) is optionally implantable.

In a third aspect, the invention is directed to a method for electrically excitable cell monitoring comprising:
i. stablishing an interaction between the monitoring device (1) of the present invention with one or more electrically excitable cells; and
ii. monitoring the measurable signals of the electrically excitable cells with the monitoring device (1) of the present invention;
wherein the method is performed *in vitro* and/or *ex-vivo.*

In a further aspect, the invention is directed to the use of the monitoring device of the present invention for cell monitoring; preferably *in vitro* and/or *ex-vivo.*

In a further aspect the invention is directed to a method of diagnosis of a disease in a subject, which method comprises:
i. stablishing an interaction between the monitoring device (1) in the present invention with one or more electrically excitable cells; and
ii. monitoring the measurable signals of the electrically excitable cells with the monitoring device (1) of the present invention;
iii. comparing the measurable signals of the electrically excitable cells with a reference value;
   - if the measurable signals of the electrically excitable cells significantly differ from the reference value; then, the subject is diagnosed with the disease;
   - if the measurable signals of the electrically excitable cells does not differ from the reference value; then, the subject is not diagnosed with the disease;
wherein the disease comprises an interruption of measurable signals transmitted among different groups of electrically excitable cells; and
wherein the method is performed *in vivo, in vitro* and/or *ex-vivo.*

In a further aspect, the invention is directed to a medical device A (2) comprising:
i. two probes (1.1) as defined in the present invention in any of its particular embodiments; wherein the two probes (1.1) are physically separated a distance of between 0.1 and 100 mm;
ii. at least one electrode or electrode array (1.2) adapted for applying an electrical pulse of a certain value; and
iii. an electrical pulse generator (1.3) adapted for generating electrical pulses of a certain value; wherein the at least one electrode or electrode array (1.2) and the electrical pulse generator (1.3) are operatively connected; and
iv. signal processing means (1.4) adapted for processing the signals of the at least one probe (1.1) and for generating an output control signal for the electrical pulse generator (1.3); wherein the two probes (1.1) are operatively connected with the signal processing means (1.4);
wherein the electrical pulse generator (1.3) and the signal processing means (1.4) are operatively connected;
wherein the at least one probe (1.1), the at least one electrode or electrode array (1.2), the electrical pulse generator (1.3) and the signal processing means (1.4) are operatively connected; and
wherein the medical device A (2) is optionally implantable.

A further aspect is directed to a method for cell monitoring and stimulating comprising
i. contacting one of the two probes (1.1) of the medical device A (2) as defined in the present invention with a group A of electrically excitable cells; wherein the electrically excitable cells generate measurable signals;
ii. contacting the at least one electrode or electrode array (1.2) of the medical device A (2) of the invention, with the group A of electrically excitable cells or with a group B of electrically excitable cells; and
wherein the method is performed *in vitro* and/or *ex-vivo.*

In a further aspect, the invention is directed to the use of the medical device A (2) of the invention for cell monitoring and stimulating *in vitro* and/or *ex-vivo.*

In a further aspect, the invention is directed to a method of treatment and/or prevention of a disease, which method comprises implanting the medical device A (2) as defined in the present invention to a subject in need of such a treatment; wherein the disease comprises a malfunction or an interruption of signal transmission among different groups of electrically excitable cells.

In a further aspect, the invention is directed to a medical device B (3) comprising:
i. at least two sets of probes; wherein each set of probes comprises two probes (1.1) as defined in any particular embodiment of the present invention; wherein in each of the set of probes, the probes (1.1) are physically separated a distance of between 0.1 and 100 mm;
ii. at least two electrodes or electrode arrays (1.2) adapted for applying an electrical pulse of a certain value;
iii. an electrical pulse generator (1.3) adapted for generating electrical pulses of a certain value; wherein the at least one electrode or electrode array (1.2) and the electrical pulse generator (1.3) are operatively connected; and
iv. signal processing means (1.4) adapted for processing the signals of the at least two sets of probes (1.1) and for generating an output control signal for the electrical stimulator (1.3); wherein the two probes (1.1) are operatively connected with the signal processing means (1.4);
wherein the electrical pulse generator (1.3) and the signal processing means (1.4) are operatively connected;
wherein the at least two sets of probes (1.1), the at least two electrodes or electrode arrays (1.2), the electrical pulse generator (1.3) and the signal processing means (1.4) are operatively connected so that the medical device B (3) is able to work bidirectionally;
wherein the medical device B (3) is optionally implantable.

In a further aspect, the invention is directed to a method for cell monitoring and stimulating comprising
i. contacting one probe (1.1) of one of the at least two sets of probes (1.1) of the medical device B (3) as defined in the invention and one of the at least two electrodes or electrode arrays (1.2) of said medical device B (3) with group A of electrically excitable cells;
ii. contacting other probe (1.1) of the at least two sets of probes (1.1) of the medical device B (3) as defined and other of the at least two electrodes or electrode arrays (1.2) of said medical device B (3) as defined with one group B of electrically excitable cells;
wherein the method is performed *in vitro* and/or *ex-vivo.*

In a further aspect, the invention is directed to the use of the medical device B (3) according for cell monitoring and stimulating *in vitro* and/or *ex-vivo.*

In a further aspect, the invention is directed to a method of treatment and/or prevention of a disease, which method comprises implanting the medical device B (3) as defined in the present invention, to a subject in need of such a treatment; wherein the disease comprises a malfunction or an interruption of signal transmission among different groups of electrically excitable cells.

### FIGURES

The accompanying figures illustrate exemplary embodiments of the invention and together with the written description serve to explain the principles of the invention.

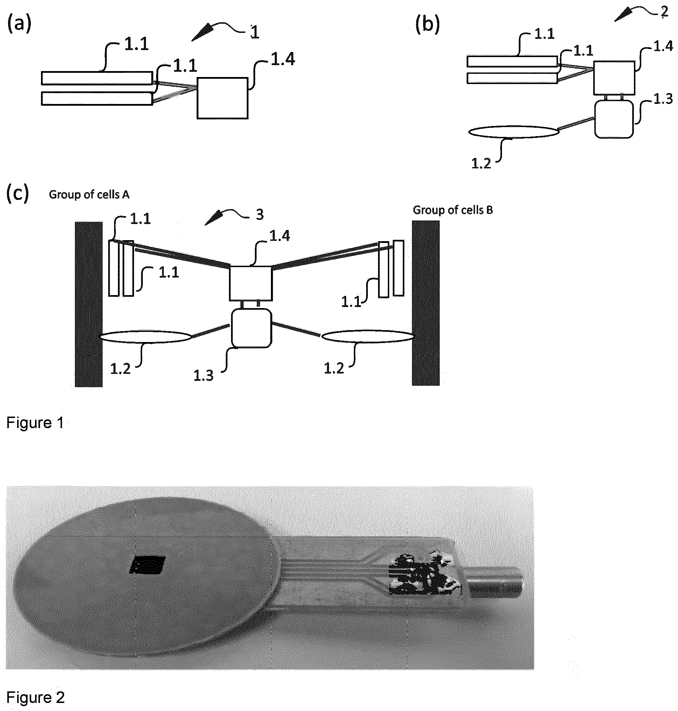

Figure 1: Schematics of (a) a monitoring device, (b) a medical device A; and (c) a medical device B.
Figure 2: Sensor.

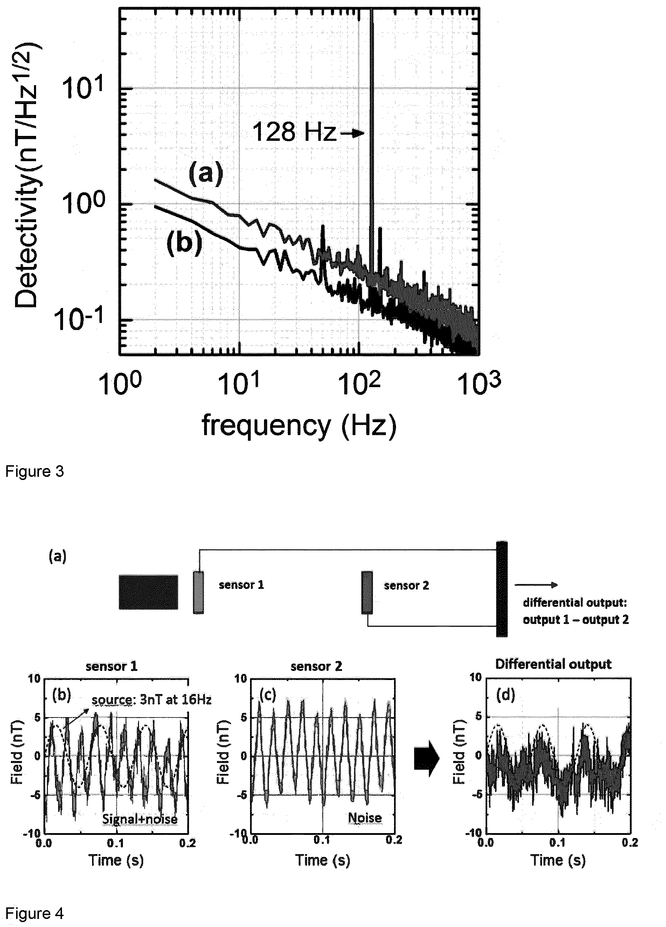

Figure 3: Graphic showing the detectivity values of the differential output of two sensors mounted in a differential configuration (line (a)) and the output of a single sensor (line (b)).
Figure 4: (a) Schematics of the differential configuration of sensors 1 and 2 and its differential output. Field values of (b) the output of a single sensor 1 placed close to a magnetic source, (c) the output of a single sensor 2 placed a further distance to the magnetic source than the single sensor 1, and (d) the differential output of two sensors mounted in a differential configuration.

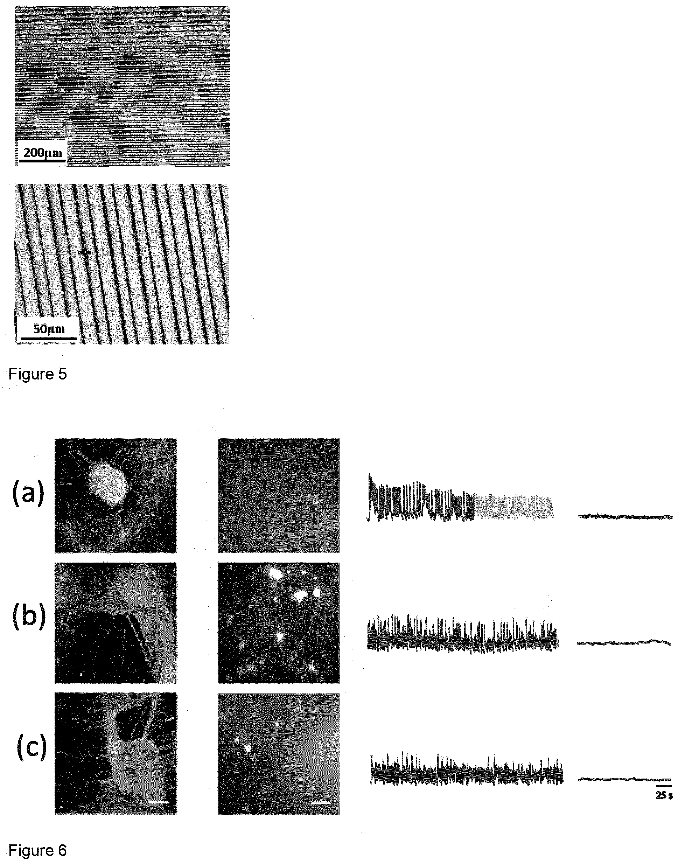

Figure 5: Scanning electron microscopy micrographs of a grooved polymeric (PS or PDMS) layer coating the magnetic sensors.
Figure 6: The left column (scale bar 500 µm) shows immunofluorescence images of neuron-specific microtubules and neurofilament H of spinal cord explants maintained *in vitro* interfaced to glass coverslips as control and two polymeric layers: (a) control, (b) grooved PDMS polymeric layer, and (c) grooved PS polymeric layer. Higher magnification fluorescence images of clusters of neurons stained with membrane-permeable Ca²⁺ dye Fluo-4 AM are shown in the second column (scale bar 50 µm). In addition, recordings of representative traces for spontaneous synaptic activity measured as live calcium fluorescence imaging as fractional amplitude increase (ΔF/F₀) in the absence (third column) and presence of tetrodotoxin (fourth column) are shown. Those recordings were performed at the premotor region in the ventral zone were spontaneous activity is more prominent.

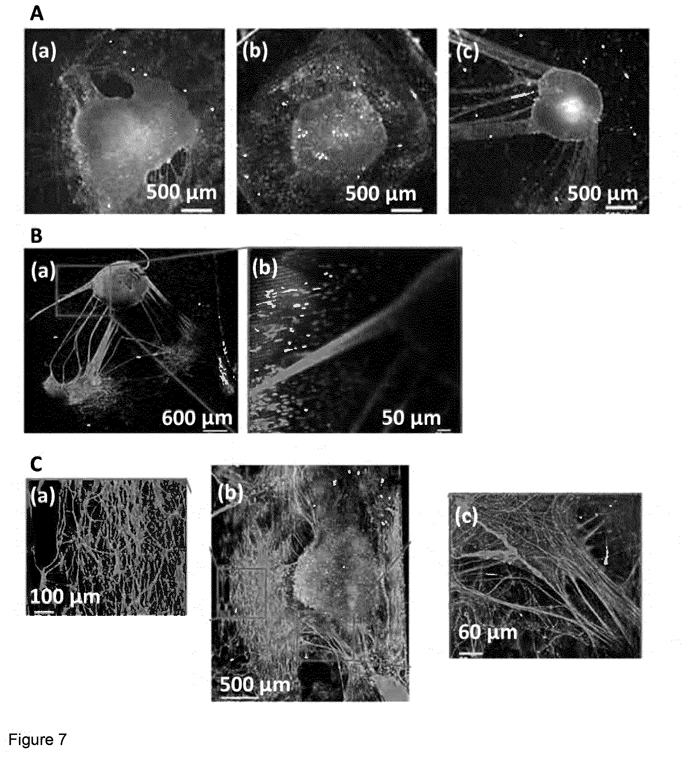

Figure 7: (A) Snapshots of (a) a representative field of neural cultures grown onto a control substrate, (b) neural cultures grown onto a grooved PDMS substrate, and (c) neural cultures grown onto a grooved PS substrate. (B) Snapshots of neural cultures grown onto a grooved PDMS substrate at different magnification. (C) Snapshots of neural cultures grown onto a grooved PS substrate at different magnification.

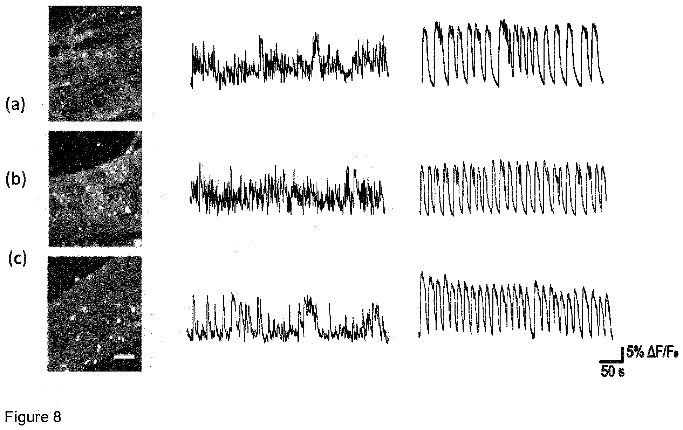

Figure 8: Snapshots of axons outgrowing the spinal slices stained with membrane-permeable Ca²⁺ dye Fluo-4 AM (scale bar 100 µm) grown on (a) control glass, (b) grooved PDMS polymeric layer, and (c) grooved PS polymeric layer. In addition, recordings of representative traces for axonal activity measured as live calcium fluorescence imaging recorded as fractional amplitude increase (ΔF/F₀) in the absence (second column), and in the presence of Bicuculline/Strychnine (third column).

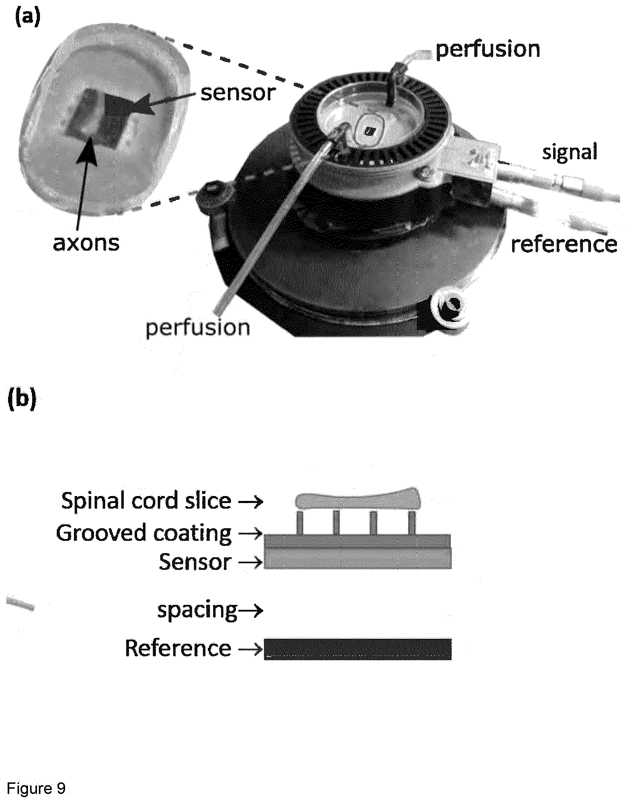

Figure 9: Diagram of the setup for the detection of neural activity on organotypic spinal slices using magnetic sensors in a differential configuration. Two magnetic sensors are placed at different distances from the spinal slice. The sensor in contact with the organotypic spinal slices is coated by a grooved polymeric layer.

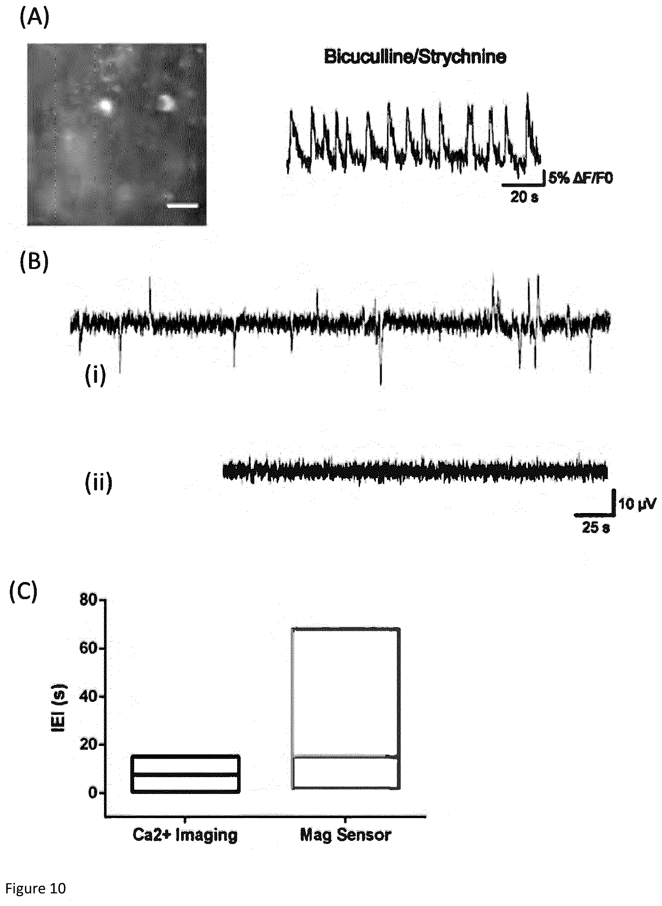

Figure 10: Results of a Ca²⁺ imaging experiment. (A) Left panel, Field image of the ventral horn of a spinal explant slice loaded with Fluo4-AM (scale bar 50 µm). Right panel, synaptic activity recordings via live calcium fluorescence imaging measured as fractional amplitude increase (ΔF/F₀) over time in the presence of Bicuculline/Strychnine. (B) Magnetic sensor test results. In particular, recordings of representative traces synaptic activity measured as magnetic measurements recorded as voltage (µV) over time (s) with the magnetic sensor of the present application in differential configuration in the presence of (i) Bicuculline/Strychnine and in the presence of (ii) Tetrodoxin. The magnetic test results were obtained during the same calcium imaging experiment above described in Figure 10(A). (C) Estimated interevent intervals (IEIs) during Bicuculline/Strychnine-induced activity obtained in the experiment with calcium imaging and with the magnetic sensors.

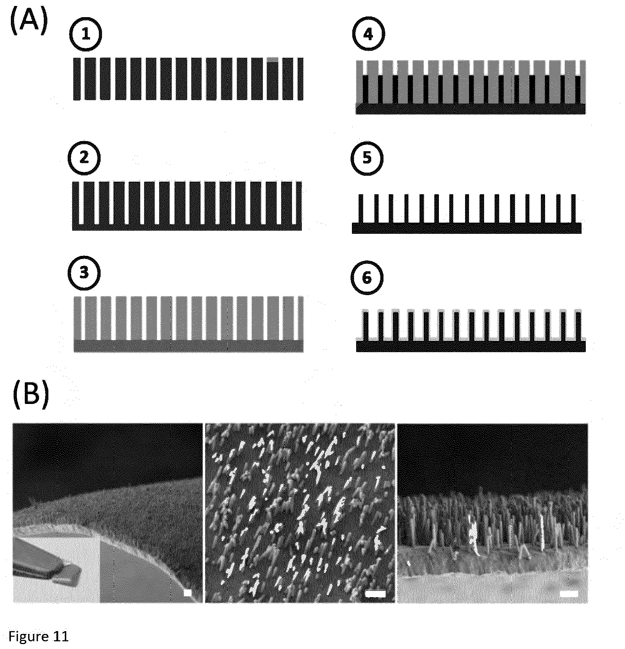

Figure 11: (A) Sketch of the nanostructured electrodes fabrication and (B) scanning electron microscopy (SEM) micrographs of the flexible nanostructured electrodes. The inset in (B) shows a photograph of a bended electrode. The scale bar is 1 micron in all images.

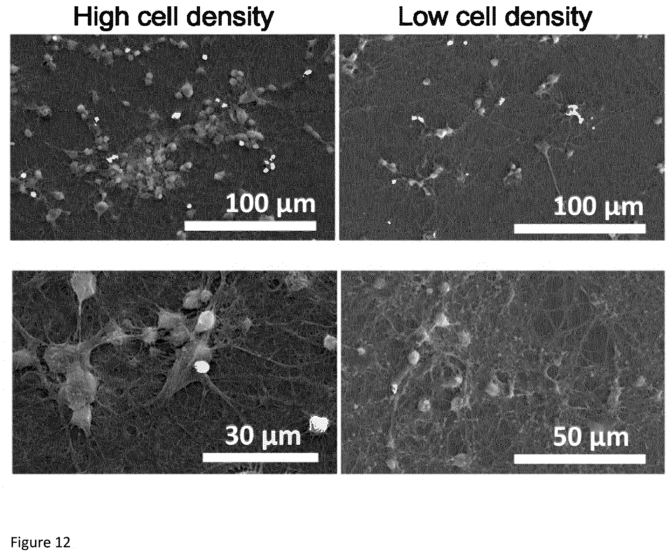

Figure 12: Representative SEM micrographs of cultures at 14 days at high- and low-density seeding conditions over PLL-coated glass coverslips (control).

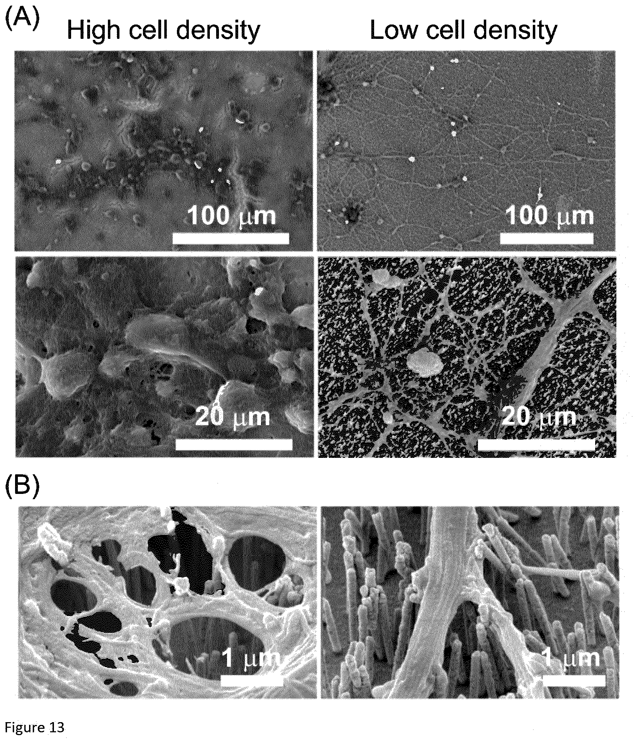

Figure 13: Representative (A) SEM and (B) field-emission scanning electron microscopy (FESEM) micrographs of cultures at 14 days at high- and low-density seeding conditions over gold nanostructured electrodes.

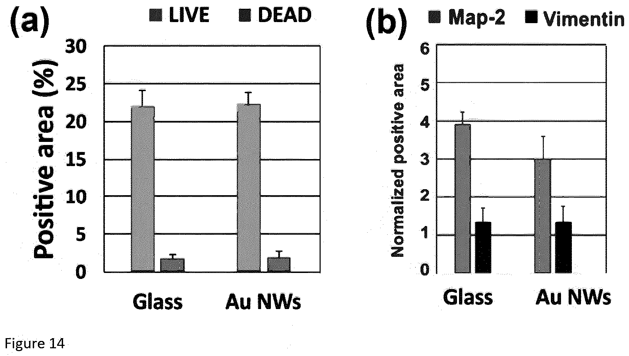

Figure 14: (a) Normalized positive area for alive and dead cells in viability studies of ENPCs cultures on the glass coverslips (control) and gold nanostructure electrodes at 14 days by confocal microscopy. Alive cells were labeled by calcein and dead cells by EthD-1. (b) Normalized positive area for neurons (map2⁺) and non-neuronal cells (vimentin⁺) in differentiation studies of ENPCs cultures on the glass coverslips (control) and gold nanostructure electrodes at 14 days by CLSM.

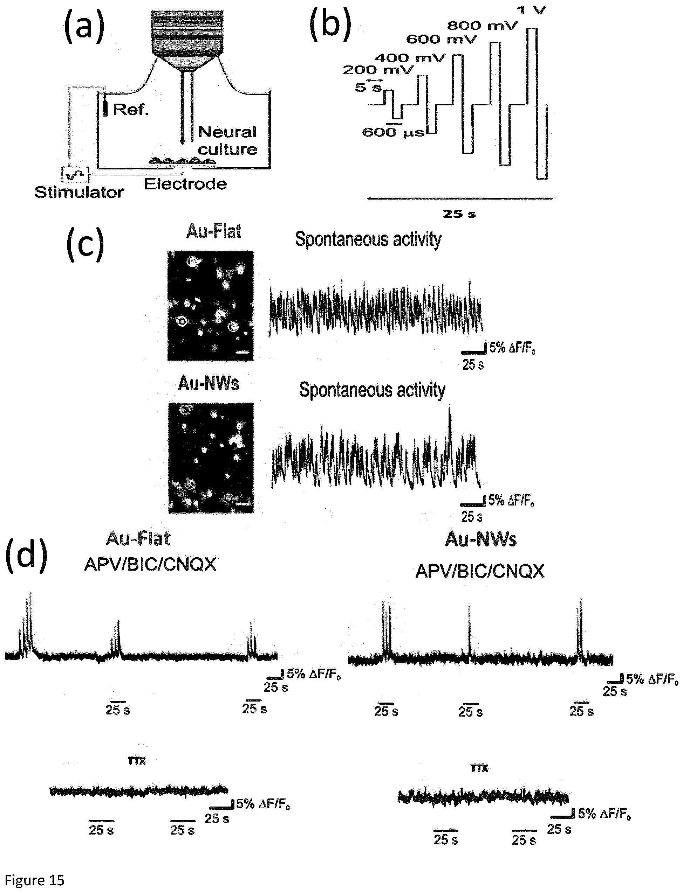

Figure 15: (a) Sketch of the experimental setting for stimulation of cultured neurons. b) The stimulation protocol: five biphasic voltage steps repeated three outdistanced times. Increasing bipolar steps from 0 to 1 V in steps of 200 mV were sent. c) Two representative snapshot of the imaged field of hippocampal dissociated cultures (scale bar = 50 µm) grown onto Au-Flat and Au-NWs electrodes are shown. A circular ROI (region of interest, circles) was drawn around spiking neurons and used to plot the calcium transient courses over the time as those on the right side of the snapshots. d) Upper panel, neural activity under pharmacological conditions such as only stimulus-evoked activity could be observed. Representative fluorescence tracings depict neuronal responses evoked by extracellular pulse stimulations (25 s-long, dashes under the traces) applied through the Au-Flat (left traces) or Au-NWs electrodes (right traces). Lower panel, neuronal cells ability to fire action potentials after voltage stimuli was totally abolished by TTX, ensuring the neuronal origin of the signals evoked in APV/BIC/CNQX condition. Calcium transients are expressed as fractional amplitude increase in the fluorescence signal (ΔF/F0, where F0 is the baseline fluorescence level and ΔF is the rise over baseline). The fluorescence scale (y-axis) is 5%, while the time scale (x-axis) is 25 s in all transients.

### DETAILED DESCRIPTION OF THE INVENTION

### Probe

In a first aspect, the invention is directed to a probe (1.1) comprising at least one sensor or sensor array adapted for monitoring a signal; wherein the probe (1.1) is biocompatible and optionally implantable; wherein the signal monitored by the at least one sensor or sensor array is a magnetic signal; preferably a magnetic field signal.

In a particular embodiment, the magnetic signal is generated by one electrically excitable cell or by a group of electrically excitable cells.

In a particular embodiment, the probe of the invention is implantable; preferably implantable *in vivo.*

In the context of the invention, the term "implantable" is directed to a device that can be an insert or fix (artificial object) in a subject such a person or animal, preferably by surgery.

In the context of the present invention, the expression "monitor" or "monitoring" a signal refers to detecting, sensing, measuring or checking a signal over a period of time.

In a particular embodiment, the probe (1.1) of the present invention further comprises a printed circuit board; in particular, wherein the printed circuit board and the at least one sensor or sensor array are operatively connected.

### Sensor

In a particular embodiment, the at least one sensor or sensor array is based on spin electronics.

In a particular embodiment, the probe (1.1) of the present invention comprises one sensor.

In a more particular embodiment, the at least one sensor or sensor array is a spintronic sensor or spintronic sensor array; preferably a sensor based on anisotropic magnetoresistance, giant magnetoresistance or tunneling magnetoresistance.

In a more particular embodiment, the at least one sensor or sensor array is at least one magnetic sensor or a magnetic sensor array; preferably is at least one magneto resistance sensor or a magneto resistance sensor array; particularly is at least one tunnel magneto resistance sensor or a tunnel magneto resistance sensor array.

In a particular embodiment, the at least one sensor or sensor array is non-cooled. In a particular embodiment, the at least one sensor or sensor array do not need additional refrigeration (i.e. is able to work at temperatures between 20 and 45 °C).

In a particular embodiment, the at least one sensor or sensor array is micron-sized; preferably the at least one sensor or sensor array has a length between 1000 microns and 1 micron; particularly a length between 600 microns and 5 microns.

In a particular embodiment, the at least one sensor has detectivity below 10 nT/Sqrt(Hz) at frequencies below 100 Hz; preferably below 1 nT/Sqrt(Hz) at frequencies below 100 Hz.

In a particular embodiment, the at least one sensor is able to detect a magnetic field; preferably a magnetic field below 20 nT at frequencies below 100 Hz; more preferably below 1 nT at frequencies below 100 Hz.

In a more particular embodiment the at least one sensor or sensor array adapted for monitoring a signal comprises two surfaces; particularly wherein one of said surfaces is a flat surface.

The authors of the present invention have observed that when tunnel magneto resistance sensors (TMR) are used in the probe of the invention, a stable monitoring signal is obtained in the temperature range of between 20 °C and 40 °C. In addition, another advantage of the present invention is the low aging deterioration of TMR sensors.

### Polymeric layer

In an embodiment, the at least one sensor or sensor array adapted for monitoring a signal of the probe of the present invention comprises two surfaces; wherein a polymeric layer is coating at least one surface of the at least one sensor or sensor array; in particular a flat surface of the at least one sensor or sensor array.

In a particular embodiment, the polymeric layer comprises:
(a) a textured surface; and
(b) a bulk portion.

In a more particular embodiment, the polymeric layer of the probe of the present invention is a thermoplastic polymeric layer.

The term "textured surface" in the context of the present invention refers to the surface of the polymeric layer of the invention comprises a plurality of protrusions; preferably micro- and/or nanometric protrusions; particularly micrometric protrusions. The protrusions in the polymeric layer of the invention may have any common regular and/or irregular shapes, such as a spherical shape, elliptical shape, cubical shape, tetrahedral shape, pyramidal shape, octahedral shape, cylindrical shape, polygonal pillar-like shape, conical shape, columnar shape, tubular shape, helical shape, funnel shape, grating shape or dendritic shape. Each of the protrusions may have the same or different shape, height, and/or width. In a particular embodiment, the protrusions have a columnar shape, a grating shape or a cylindrical shape. In another particular embodiment, the protrusions have a pillar shape. In another particular embodiment, the protrusions have a grating shape. In another particular embodiment, the polymeric layer is a grooved polymeric layer.

In an embodiment, the textured surface comprises a plurality of protrusions, preferably with a grating shape.

In one embodiment, the protrusions of the textured surface present a height of between 5 nm and 1000 µm, preferably between 5 nm and 100 µm, more preferably of between 10 nm and 20 µm, more preferably between 50 nm and 10 µm, more preferably of between 100 nm and 5 µm, even more preferably between 200 nm and 3 µm, even more preferably between 250 nm and 2000 nm. In another preferred embodiment, the protrusions present a height of between 1 µm and 12 µm.

In one embodiment, the protrusions present a width between 5 nm and 10000 µm, preferably between 5 nm and 1000 µm, more preferably between 10 nm and 20 µm, more preferably of between 50 nm and 15 µm; even more preferably of about 10 µm. In another preferred embodiment, the protrusions in the textured surface present a width of between 5 µm and 15 µm. In the context of the present invention, the expression width may be considered as diameter.

In one embodiment, the protrusions present an interval between the protrusions or pitch of between 5 nm and 1000 µm, preferably between 5 nm and 100 µm, more preferably of between 10 nm and 30 µm, even more preferably of about 20 µm. The intervals between the protrusions may also be the same or different; preferably the same.

In a particular embodiment, the protrusions have a grating shape and present a height of between 1 µm and 10 µm, a width of between 1 µm and 20 µm, and an interval between the protrusions of between 1 µm and 30 µm. In another particular embodiment, the protrusions present a height of 5 µm, a width of 10 µm and an interval between protrusions of 20 µm.

The polymer in the polymeric layer may be a thermoplastic polymer. In a particular embodiment, the thermoplastic polymer is selected from poly(methyl methacrylate) (PMMA), polycarbonate (PC), polystyrene (PS), polydimethylsiloxane (PDMS), cellulose acetate (CA) and mixtures thereof. In another particular embodiment the thermoplastic polymer is a polyolefin selected from polypropylene (PP), polyethylene (PE), and mixtures thereof. In another particular embodiment the thermoplastic polymer is selected from polylactic acid (PLA), polycaprolactone (PCL), polyhydroxyalkanoate (PHA), polyhydroxybutyrate (PHB), polyethylene adipate (PEA), polybutylene succinate (PBS), poly(3-hydroxybutyrate-co-3-hydroxyvalerate) (PHBV), polytrimethylene terephthalate (PTT), polyethylene naphthalate (PEN) and mixtures thereof. In a preferred embodiment the thermoplastic polymer is selected from polyglycolide (PGA), poly(propylenefumarate) (PPF), polycyanoacrylate (PCA), polycaprolactone (PCL), poly(glycerol sebacate) (PGS), poly(glycerol sebacate acrylate) (PGSA), polyvinylidenefuoride (PVDF), polyvinylidene chloride (PVDC), polyethylene terephthalate (PET), polybutylene therephtalate (PBT), polyphenylene oxide (PPO), polyvinyl chloride (PVC), cellulose acetate (CA), cyclic olefin copolymer (COC), ethylene vinyl acetate (EVA), ethylene vinyl alcohol, (EVOH), polyethersulfone (PES), chlorinated poly ethylene (CPE), poly 3-hydroxybutyrate (P3HB), polybutylene adipate (PBA), polyethylene adipate (PEA), polybutylene succinate (PBS), polyphenylene sulfide (PPS), polysulfone (PSU), polytrimethylene terephthalate (PTT), polyurethane (PU), polyvinyl acetate (PVAc), styrene acrylonitrile (SAN), and mixtures thereof. In another particular embodiment the thermoplastic polymer is selected from polyetherketones (PEEK), polyvinylalcohol (PVA), polyhydroxyethylmethacrylate polyvinylalcohol (PHMM-PVA), polyhydroxyethylmethacrylate (PHEMA), poly(N-isopropylacrylamide) (PNIPAAm) and mixtures thereof. In a preferred embodiment the polymer in the polymeric matrix is polystyrene (PS), polydimethylsiloxane (PDMS), polycarbonate (PC), polyethylene (PE), polypropylene (PP), poly(methyl methacrylate) (PMMA) and mixtures thereof; preferably polystyrene (PS) or polydimethylsiloxane (PDMS); more preferably polydimethylsiloxane (PDMS).

In a preferred embodiment, the thermoplastic polymer is selected from poly(methyl methacrylate) (PMMA), polycarbonate (PC), polystyrene (PS), polydimethylsiloxane (PDMS), polypropylene (PP), polyethylene (PE), polyglycolide (PGA), poly(propylenefumarate) (PPF), polycyanoacrylate (PCA), polycaprolactone (PCL), poly(glycerol sebacate) (PGS), poly(glycerol sebacate acrylate) (PGSA), polyvinylidenefuoride (PVDF), polyvinylidene chloride (PVDC), polyethylene terephthalate (PET), polybutylene therephtalate (PBT), polyphenylene oxide (PPO), polyvinyl chloride (PVC), cellulose acetate (CA), cyclic olefin copolymer (COC), ethylene vinyl acetate (EVA), ethylene vinyl alcohol, (EVOH), polytetrafluoroethylene (PTFE), fluorinated ethylene propylene (FEP), perfluoroalkoxy (PEA), ethylene tetrafluoroethylene (ETFE), polyethersulfone (PES), chlorinated poly ethylene (CPE), polylactic acid (PLA), poly 3-hydroxybutyrate (P3HB), polybutylene adipate (PBA), polyethylene adipate (PEA), polybutylene succinate (PBS), polyphenylene sulfide (PPS), polysulfone (PSU), polytrimethylene terephthalate (PTT), polyurethane (PU), polyvinyl acetate (PVA), polyvinylidene chloride (PVDC), styrene acrylonitrile (SAN), polyetherketones (PEEK), polyvinylalcohol (PVA), polyhydroxyethylmethacrylate (PHEMA), poly(N-isopropylacrylamide) (PNIPAAm) and mixtures thereof.

In another particular embodiment, the probe of the invention is part of a device; preferably part of a medical device; more preferably part of an implantable medical device; even more preferably part of a surgically implantable medical device.

The probe may be used as part of any other system, method or device that would benefit from stable, non-destructive interaction with a cell; preferably with an electrically excitable cell; more preferably with an electrically excitable cell that is able to emit magnetic signals. Similarly, the probes described herein may be used to form part of a biological interface for a medical device (such as an implant, prosthetic, or the like). In another particular embodiment the probe is implantable, preferably *in vivo.*

The authors of the present invention have observed that when a polymeric layer is coating at least one surface of the at least one sensor or sensor array of the probe of the present invention the interaction of the probe with the source of signals (such as electrically excitable cells) is maximized.

### Monitoring device

In a second aspect, the invention is directed to a monitoring device (1) comprising:
i. two probes (1.1) as defined in the present invention in any of its particular embodiments; wherein the two probes (1.1) are physically separated a distance of between 0.1 and 100 mm; and
ii. signal processing means (1.4) adapted for processing the signals of the probes;
wherein the two probes (1.1), and the signal processing means (1.4) are operatively connected; and wherein the monitoring device (1) is optionally implantable.

The authors of the present invention have found that when the two probes of the monitoring device (1) of the invention are mounted in a differential configuration (i.e. one probe is placed closer to the magnetic source of interest than the other), while the probe placed closer to the magnetic source of interest monitors the signals produced by said magnetic source of interest and noise, the probe situated further from the magnetic source of interest monitors noise not related to the signals produced by the magnetic source of interest. Then, the signals monitored might be subtracted and thus, the noise reduced in the output (i.e. by an amplifier).

In a particular embodiment, the two probes of the monitoring device (1) of the invention are mounted in a differential configuration between them.

In a particular embodiment, the two probes of the monitoring device (1) of the invention are physically separated a distance of between 0.1 mm and 10 mm; preferably of between 1 mm and 10 mm; more preferably a distance of between 2 mm and 5 mm.

In a more particular embodiment, a first probe is placed closer to the magnetic field source of interest than the second sensor probe, which is placed at a further distance from the source of interest.

In a particular embodiment, the signal processing means are adapted for processing the signals monitored by the probes; particularly the signals received and transmitted by the probes to the signal processing means.

In a particular embodiment, the monitoring device (1) of the invention further comprises an amplifier; wherein the two probes (1.1), the amplifier and the signal processing means (1.4) are operatively connected.

In a particular embodiment, the signal processing means is a processor. As will be appreciated by one of skill in the art, any device according to the invention may contain a signal processing means containing instructions which cause the processor (computing system) to carry out the method according to the invention. The processor may take the form of an entirely hardware embodiment or an embodiment combining software and hardware aspects. Furthermore, the present invention may include a computer program product on a computer-usable storage medium having computer-usable program code means embodied in the medium. Any suitable computer readable medium may be utilized including hard disks, CD-ROMs, optical storage devices, or magnetic storage devices. The computer-usable or computer-readable medium may be or include, for example, but not limited to, an electronic, magnetic, optical, electromagnetic, infrared, or semiconductor system, apparatus, device, or propagation medium. More specific examples (a non-exhaustive list) of the computer readable medium would include the following: an electrical connection having one or more wires, a portable computer diskette, a random access memory (RAM), a read-only memory (ROM), an erasable programmable read-only memory (EPROM or Flash memory), an optical fiber, a portable compact disc read-only memory (CD-ROM), a CD ROM, a DVD (digital video disk), or other electronic storage medium. Note that the computer-usable or computer-readable medium could even be paper or another. Suitable medium upon which the program is printed, as the program can be electronically captured, via, for instance, optical scanning of the paper or other medium, then compiled, interpreted or otherwise processed in a suitable manner if necessary, and then stored in a computer memory.

Computer program code for carrying out operations of the present invention may be written in an object oriented programming language such as JavaE, Smalltalk or C++. However, the computer program code for carrying out operations of the present invention may also be written in conventional procedural programming languages, such as the "C" or FORTRAN programming language or even assembly language. The program code may execute entirely on the user's computer, partly on the user's computer, as a stand-alone software package, partly on the user's computer and partly on a remote computer or entirely on the remote computer. In the latter scenario, the remote computer may be connected to the user's computer through a local area network (LAN) or a wide area network (WAN), or the connection may be made to an external computer (for example, through the internet using an internet service provider).

In a particular embodiment, the monitoring device (1) of the invention is implantable; preferably surgically implantable; more preferably implantable *in vivo.*

The monitoring device works monitoring the signals (particularly magnetic signals) produced by a magnetic source of interest by monitoring signals; preferably magnetic signals and transmitting the signals to the signal processing means (1.4) of the monitoring device (1).

### Method

In a third aspect, the invention is directed to a method for electrically excitable cell monitoring comprising:
i. stablishing an interaction between the monitoring device (1) of the present invention with one or more electrically excitable cells;
ii. monitoring the measurable signals of the electrically excitable cells with the monitoring device (1) of the present invention;
wherein the method is performed *in vitro* and/or *ex-vivo.*

In a particular embodiment, the method is performed *in vivo, in vitro* and/or *ex-vivo;* preferably *in vivo.*

In a more particular embodiment, the interaction between the monitoring device (1) of the present invention with one or more electrically excitable cells is between:
- one of the two probes (1.1) of the monitoring device (1) of the present invention; and
- one or more electrically excitable cells.

In the context of the present invention the term "interaction" is directed to a physical contact or to non-physical contact between the monitoring device (such as between at least one probe of the monitoring device) and the one or more electrically excitable cells. Said interaction allows the detection, measurement or recording of the measurable signals emitted by the electrically excitable cells.

In a particular embodiment, the interaction of the method of the invention is a physical contact.

In a more particular embodiment, at least one surface of the at least one sensor or sensor array of the probe of the monitoring device of the present invention is coated with a polymeric layer and the interaction is a physical contact between said polymeric layer and the one or more electrically excitable cells.

In a particular embodiment, the monitoring of the measureable signals comprises the steps of: (a) detecting and (b) processing said measurable signals within the monitoring device (1).

In the context of the present invention, the expression "electrically excitable cells" is understood as electrically active cell types that are able to generate an action potential in response to a depolarisation of the membrane potential above an excitation threshold.

In a particular embodiment, "electrically excitable cells" may be selected from neuronal, cardiac or muscle cells; preferably neural and/or cardiac cells; more preferably neural cells.

In a particular embodiment, electrically excitable cells are one or more neurons; preferably a neural cell network; more preferably an active neural cell network.

In the context of the present invention, the term "neural cell" is meant to include cells from the central nervous system (CNS) or the peripheral nervous system (PNS). Exemplary neural cells of the CNS are found in the gray matter of the spinal cord and the brain and exemplary neural cells of the PNS are found in the dorsal root ganglia. The expression "neural cell network" or "neural network" is understood as comprising neural circuits, i.e. a population of neurons interconnected by synapses; spontaneously active neural cell network, displaying spontaneous electrical and synaptic activity. Thus, synchronized patterned activity emerges when the efficiency of the network connections, synapses, is high.

The "activity" of the neural cell network may be measured by for example calcium imaging characterization techniques known in the art, i.e. by loading the neural cell network with a Ca²⁺ dye and imaging the intracellular calcium activity of the cell network; preferably using fluorescence live imaging. In addition, the "activity" of the neural cell network may be identified as from neuronal origin by removing the action potentials using drugs (for example by using tetrodotoxin [TTX]). In addition, the "activity" of the neural cell network generates ionic flows and thereby both magnetic fields and electric potential differences. In particular, the magnetic fields generated may be measured by at least one sensor or sensor array of the probe (1.1) of the present invention (for example by using a magnetoresistive sensor).

In a particular embodiment, the electrically excitable cells are able to generate measurable signals; particularly magnetic signals; more particularly currents (cell undergoes a shift in electric charge distribution such as in depolarization processes) that create magnetic fields, those magnetic fields might be measured in the present invention by at least one sensor or sensor array of the probe (1.1) of the present invention (for example by using a magnetoresistive sensor).

In a particular embodiment, the "measurable signals" are magnetic signals; preferably magnetic field signals.

In a particular embodiment, the "measurable signals" of the invention are magnetic signals; preferably magnetic field signals in the order of several nT; preferably of between -30 and +30 nT; more preferably of between -20 and +20 nT; even more preferably of between -10 and +10 nT.

### Use of the monitoring device

The monitoring device of the present invention in any of its particular embodiments can be used for cell monitoring; preferably for electrically excitable cell monitoring *in vitro* and/or *ex-vivo;* even more preferably for electrically excitable cell monitoring *in vitro* and/or *ex-vivo* .

In a particular embodiment, the use of the monitoring device of the present invention is for electrically excitable cell monitoring; preferably *in vitro* and/or *ex-vivo.*

In a particular embodiment, the use of the monitoring device of the present invention is *in vivo.*

### Method of diagnosis

In a further aspect the invention is directed to a method of diagnosis of a disease in a subject, which method comprises:
i. stablishing an interaction between the monitoring device (1) of the present invention with one or more electrically excitable cells; and
ii. monitoring the measurable signals of the electrically excitable cells with the monitoring device (1) of the present invention;
iii. comparing the measurable signals of the electrically excitable cells with a reference value so that;
   - if the measurable signals of the electrically excitable cells significantly differs from the reference value; then the subject is diagnosed with the disease;
   - if the measurable signals of the electrically excitable cells does not differ from the reference value; then the subject is not diagnosed with the disease;
   wherein the disease comprises an interruption of measurable signals transmitted among different groups of electrically excitable cells; and
   wherein the method is performed *in vivo, in vitro* and/or *ex-vivo.*

In a particular embodiment, the disease of the method of diagnosis of a disease is selected from any of chronic pain, epilepsy, Parkinson's disease, sacral nerve pelvic disorders and incontinence, spinal cord traumatic disorders or injuries and spinal cord ischemic disorders or injuries (*e.g*. angina, peripheral vascular disease); particularly for traumatic and/or ischemic spinal cord injury (SCI).

In the context of the present invention, the term "diagnosis" relates to the ability to discriminate if the measurable signals of the electrically excitable cells of a patient (the subject) significantly differs from a reference value, when applied a method as disclosed herein. This detection as it is understood by one skilled in the art is not intended to be 100% correct for all the samples. However, it requires that a statistically significant number of measurable signals analyzed are classified correctly. The amount that is statistically significant can be set by an expert in the field by using different statistical tools, for example, but not limited by the determination of confidence intervals, p value determination, Student's t test and discriminating function Fisher. Preferably, the confidence intervals are at least 90%, at least 95%, at least 97%, at least 98% or less than 99%. Preferably, the p value is less than 0.05, 0.01, 0.005 or 0.0001. Preferably, the present invention can correctly detect a disease in at least 60%, at least 70%, by at least 80%, or at least 90% of the subjects of a particular group or population tested.

The term "subject", or "individual" or "animal" or "patient" includes any subject, particularly a mammalian subject, for whom therapy is desired. Mammalian subjects include humans, domestic animals, farm animals, and zoo or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "reference value", as used herein, relates to a predetermined criteria used as a reference for evaluating the values or data obtained from monitoring the measurable signals of the electrically excitable cells from a subject. The reference value or reference level can be an absolute value; a relative value; a value that has an upper or a lower limit; a range of values; an average value; a median value; a mean value; or a value as compared to a particular control or baseline value. A reference value can be based on an individual signal of the electrically excitable cells of the subject, such as for example, at an earlier point in time. The reference value can be based on a large number of measurable signals, such as from population of subjects of the chronological age matched group, or based on a pool of samples including or excluding the sample to be tested.

The reference value used in the diagnostic method of the invention can be optimized in order to obtain a desired specificity and sensitivity.

### Medical devices

In a further aspect, the invention is directed to a medical device A (2) comprising:
i. two probes (1.1) as defined in the present invention in any of its particular embodiments; wherein the two probes (1.1) are physically separated a distance of between 1 and 10 mm;
ii. at least one electrode or electrode array (1.2) adapted for applying an electrical pulse of a certain value;
iii. an electrical pulse generator (1.3) adapted for generating electrical pulses of a certain value; wherein the at least one electrode or electrode array (1.2) and the electrical pulse generator (1.3) are operatively connected; and
iv. a signal processing means (1.4) adapted for processing the signals of the at least one probe (1.1) and for generating an output control signal for the electrical pulse generator (1.3); wherein the two probes (1.1) are operatively connected with the signal processing means (1.4);
wherein the electrical pulse generator (1.3) and the signal processing means (1.4) are operatively connected;
wherein the at least one probe (1.1), the at least one electrode or electrode array (1.2), the electrical pulse generator (1.3) and the signal processing means (1.4) are operatively connected; and
wherein the medical device A (2) is optionally implantable.

In a further aspect, the invention is directed to a medical device B (3) comprising:
i. at least two sets of probes; wherein each set of probes comprises two probes (1.1) as defined in any particular embodiment of the present invention; wherein in each set of probes, the probes (1.1) are physically separated a distance of between 1 and 10 mm;
ii. at least two electrodes or electrode array (1.2) adapted for applying an electrical pulse of a certain value;
iii. an electrical pulse generator (1.3) adapted for generating electrical pulses of a certain value; wherein the at least one electrode or electrode array (1.2) and the electrical pulse generator (1.3) are operatively connected; and
iv. a signal processing means (1.4) adapted for processing the signals of the at least two sets of probes (1.1) and for generating an output control signal for the electrical stimulator (1.3); wherein the two probes (1.1) are operatively connected with the signal processing means (1.4);
wherein the electrical pulse generator (1.3) and the signal processing means (1.4) are operatively connected;
wherein the at least two sets of probes (1.1), the at least two electrodes or electrode arrays (1.2), the electrical pulse generator (1.3) and the signal processing means (1.4) are operatively connected so that the medical device B (3) is able to work bidirectionally; and wherein the medical device B (3) is optionally implantable.

In the context of the present invention a medical device "A" and a medical device "B" can be understood as a "first" medical device and a "second" medical device. In an alternative particular embodiment, the monitoring device (1), and/or the medical device A, may have just one probe (1.1) as defined in the present invention in any of its particular embodiments instead of two probes (1.1.). In another particular embodiment, each of the set of probes of the medical device B (3) may have just one probe (1.1) as defined in the present invention in any of its particular embodiments instead of two probes (1.1.).

### Electrodes

The electrodes or electrode arrays adapted for applying an electrical pulse of a certain value used in the medical devices A and/or B of the present invention might be any of those available in the art. In another particular embodiment, electrodes or electrode arrays adapted for applying an electrical pulse of a certain value used in the medical devices A and/or B of the present invention is an electrode of an electrode pair as known in the art. In a more particular embodiment, electrodes or electrode arrays adapted for applying an electrical pulse of a certain value used in the medical devices A and/or B of the present invention is the anode or the cathode; preferably the anode.

For example, electrodes or electrode arrays adapted for applying an electrical pulse of a certain value used in the medical devices A and/or B of the present invention may be the anode having a positive potential with respect to the cathode. The polarity electrodes or electrode arrays adapted for applying an electrical pulse of a certain value used in the medical devices A and/or B of the present invention may be changed (reversed relative to each other, or increased or decreased). The electrodes or electrode arrays adapted for applying an electrical pulse of a certain value used in the medical devices A and/or B of the present invention can therefore be used for electrical cell stimulation to promote electrical or electro-physiological activity (e.g. through changes in the cell membrane potential of electrically excitable cells). Such electrical conditions may occur under physiological conditions, *in vivo, in vitro, ex-vivo* or in the presence of known or unknown drugs; preferably *in vivo.*

In a particular embodiment, the electrodes or electrode arrays adapted for applying an electrical pulse of a certain value used in the medical devices A and/or B of the present invention are electrically conductive. In a more particular embodiment, the electrodes or electrode arrays adapted for applying an electrical pulse of a certain value used in the medical devices A and/or B of the present invention are able to pass current with applied voltage through either faradic or capacitive mechanisms.

In a particular embodiment, the medical devices of the present invention further comprises a ground output, preferably an electrode as a ground output, more preferably a Ag/AgCI pellet electrode as a ground output.

In a particular embodiment, the electrodes or electrode arrays adapted for applying an electrical pulse of a certain value used in the medical devices A and/or B of the present invention comprise:
a) a textured surface with a plurality of protrusions, and
b) a bulk portion.

In a more particular embodiment the bulk portion is a sheet; preferably a flexible sheet; more preferably a metallic flexible sheet; wherein the metal of the metallic sheet is selected from Au, Pt, Co, Fe, Ni and/or combinations thereof; preferably wherein the metal is Au or Pt; more particularly wherein the metal is Au.

In a more particular embodiment the textured surface with a plurality of protrusions comprises nanowires. In the context of the present invention the term "nanowire" is understood as structures that have a thickness or diameter below one micron and an unconstrained length. In a particular embodiment, the nanowires are metallic or metallic oxide nanowires; more particularly metallic nanowires; even more particularly metallic nanowires wherein the metal is selected from Au, Pt, Co, Fe, Ni and/or combinations therefore; preferably wherein the metal is Au, Ni or Pt; more particularly wherein the metal is Au or Ni; even more particularly Au.

In a particular embodiment, the nanowires have a core-shell structure; particularly a metallic or a metallic/metallic oxide core-shell structure; particularly Ni coated with Au or Pt; preferably Au. In a particular embodiment the nanowires have a multicore or multilayered structure; particularly a metallic or a metallic/metallic oxide structure. In a more particular embodiment, the nanowires have a diameter of between 1 and 500 nm, preferably of between 20 and 400 nm; more preferably of between 30 and 300 nm. In the context of the present invention the diameter of the nanowires refers to a mean diameter measure by measuring a representative sample of nanowires by electron microscopy techniques.

In a more particular embodiment, the electrodes or electrode arrays of the invention are obtainable by a template-assisted electrodeposition method on a metal sheet; preferably wherein said method comprises a step of a metal potentiostatic deposition on a template; preferably a polycarbonate template followed by a step wherein the template is dissolved. In a particular embodiment the metal of the sheet is the same as the metal of the nanowires; preferably is Au. In another particular embodiment, the metal of the sheet is Au and the metal of the nanowires is Au or Ni. In a more particular embodiment, the electrodes or electrode arrays of the invention are metallic.

In a particular embodiment, the electrodes or electrode arrays adapted for applying an electrical pulse of a certain value used in the medical devices A and/or B of the present invention comprise:
a) a textured surface with a plurality of nanowires, and
b) a bulk portion, wherein the bulk portion is a sheet; preferably a metallic sheet.

In an embodiment, the metallic sheet has a thickness below 3, 2 or 1.5 microns. In a particular embodiment, the metallic sheet is flexible.

In a particular embodiment, the electrodes or electrode arrays adapted for applying an electrical pulse of a certain value used in the medical devices A and/or B of the present invention comprise:
a) a textured surface with a plurality of metallic or metallic oxide nanowires, and
b) a bulk portion, wherein the bulk portion is a metallic sheet.

In a more particular embodiment, the electrodes or electrode arrays adapted for applying an electrical pulse of a certain value used in the medical devices A and/or B of the present invention comprise:
a) a textured surface with a plurality of metallic nanowires, and
b) a bulk portion, wherein the bulk portion is a metallic sheet; wherein either the metallic nanowires and/or the metallic sheet comprise Au or Pt; particularly the metallic nanowires and/or the metallic sheet comprise a core of Ni coated with Au or Pt; or the metallic nanowires and/or the metallic sheet consist of Au or Pt.

In an even more particular embodiment, the nanowires are on top of the bulk portion.

In a more particular embodiment, the electrodes or electrode arrays of the present invention further comprise an electrical contact such as a platinum wire.

In another particular embodiment, the electrodes or electrode arrays adapted for applying an electrical pulse of a certain value used in the medical devices A and/or B of the present invention comprise:
a) a textured surface with a plurality of protrusions, and
b) a bulk portion;
wherein both the textured surface and/or the bulk portion comprise a polymeric matrix; and wherein both the textured surface and/or the bulk portion comprise particles having a mean size of between 1 nm and 1000 µm, wherein said particles are homogeneously distributed among the bulk portion and the textured surface; and wherein the particles are electrically conductive; and optionally, an electrical connection comprising a conductive wire.

In the context of the present invention, an "electrical connection" is understood as known in the art, for example, as a metal film or contact deposited by for example sputtering the metal onto any of the surfaces of the substrate of the present invention by means in the art. In addition, "a conductive wire" may be an electrically conductive wire such as a metallic wire as known in the art. Preferably, said electrical connection and/or wire may comprise metals as Au, Ag, Cu, Pt and/or combinations thereof. In a particular embodiment, the electrical connection is in the base of the bulk portion.

In a particular embodiment, the particles of the polymeric layer of the present invention are distributed in the polymeric matrix; preferably homogeneously distributed in the polymeric matrix among the bulk portion and the textured surface of the polymeric layer of the present invention.

In a preferred embodiment, the particles of the polymeric matrix are embedded in the polymeric matrix; preferably homogeneously embedded and/or distributed in the polymeric matrix. In a particular embodiment, the polymeric matrix comprises between 0.1 and 80 wt% of particles; preferably between 0.1 and 40 wt% of particles; more preferably between 0.1 and 30 wt%. In a particular embodiment, the particles of the polymeric matrix present a mean size of between 1 nm and 1000 µm. In the context of the present invention by the term "mean size" it is understood the average size of the largest dimension of each particle of the polymeric layer. The mean size of the particles may be calculated from the size values of a statistically significant sample measured by methods known in the art, for example by electron microscopy. Preferably, the particles of the polymeric layer of the invention present a mean size of between 2 nm and 100 µm; more preferably, between 10 nm and 50 µm; more preferably between 20 nm and 30 µm; even more preferably from 10 nm and 15 µm. In a preferred embodiment, the particles of the polymeric layer of the invention present a mean size of between 100 nm and 10 µm; more preferably of between 200 nm and 5 µm; more preferably of between 300 nm and 3 µm; even more preferably of between 500 nm and 2 µm. In a more preferred embodiment, the particles of the polymeric layer of the invention present a mean size of between 700 nm and 1 µm; preferably between 800 nm and 900 nm. In another preferred embodiment, the particles of the polymeric layer present a mean size of between 1 nm and 1 µm; preferably between 10 nm and 900 nm. The particles of the polymeric matrix may have any common regular and/or irregular shape, such as a spherical shape, elliptical shape, cubical shape, tetrahedral shape, pyramidal shape, octahedral shape, cylindrical shape, polygonal pillar-like shape, conical shape, columnar shape, tubular shape, helical shape, funnel shape, wire shape or dendritic shape. In addition, a mixture of particles with different shapes may be also used, for example tubular and spherically shaped particles. In a particular embodiment, the particles in the polymeric matrix are selected from carbon based particles, metal particles, and mixtures thereof.

### Electrical pulse generator

In the context of the invention, the expression "electrical pulse generator" is directed to a device that is able to generate and deliver one or more unipolar pulses of varying strength, such as varying voltage (V), and duration (s) with varying frequency (Hz) to the probe of the present invention. The electrical pulse generator of the medical devices of the present invention may deliver one or more unipolar pulses or a voltage pulse train.

In a particular embodiment, the voltage pulse train delivered is between 1 and 50 s length and comprises pulses of between 1 and 10 ms duration being the pulses separated among them for a time interval of between 1 and 50 s; preferably, the voltage pulse train delivered is between 10 and 40 s length and comprises pulses of between 4 and 6 ms of duration and the pulses are separated among them for an interval of between 10 and 30 s.

In a more particular embodiment, the electrical pulses generated by the stimulation device of the present invention have intensities of between 0.01 and 100 V; preferably between 0.1 and 50 V; more preferably between 0.1 and 10 V.

The electrical pulse generator of the medical devices of the present invention may be a commercial pulse generator, for example a Digitimer^{™} pulse generator. The electrical pulse generator of the present invention may be triggered by a function generator. In addition, the current on the stimulating electrodes may be recorded. The threshold for excitation of the cell may be related to the geometry and surface of the electrodes as well as to the orientation of the cell in the electric field.

In a particular embodiment, when the medical devices of the present invention are in use as such or as part of another device, the at least two electrodes or electrode array are in contact with the surface of one or several cells of the electrically excitable cells.

The signal processing means are adapted for processing the signals of the at least one probe (1.1) and for generating an output control signal for the electrical pulse generator (1.3). In a particular embodiment, the signal processing means comprise a control unit in data communication with the at least one probe and with the electrical pulse generator.

In a particular embodiment, the medical devices A and/or B are implantable, preferably surgically implantable medical devices; more preferably surgically implantable *in vivo.*

In a particular embodiment, the medical devices A and/or B are implanted or catheter based; preferably permanently implanted; more preferably *in vivo.*

### Medical device A

In a more particular embodiment, the medical device A is adapted for connecting at least two groups of electrically excitable cells A and B; wherein the connection between the at least two groups of electrically excitable cells A and B is unidirectional and comprises the following steps:
i. detecting the signals generated by the group A of electrically excitable cells with one of the two probes (1.1) of the medical device A (2);
ii. processing said signals and generating an output control signal with the signal processing means (1.4) of the medical device A (2); and
iii. electrically stimulating the group B of electrically excitable cells with the electrical pulse generator (1.3) and through the at least one electrode or electrode array (1.2) of the medical device A (2) respectively; wherein the at least one electrode or electrode array (1.2) is in contact with one or more cells of the group B of electrically excitable cells.

### Medical device B

The medical device B (3) of the present invention is able to work bidirectionally. In the context of the present invention, the term "bidirectionally" refers to work in both directions with the signal source such as electrically excitable cells. For example, the medical device B is able to connect two groups of cells being able to reestablish signal transmission among different groups of electrically excitable cells. In a particular embodiment, the medical device B is able to connect two groups of electrically excitable cells. In a more particular embodiment, the medical device B is adapted for connecting at least two groups of electrically excitable cells; in particular, is adapted for transmit signals between at least two groups of electrically excitable cells.

In a more particular embodiment, the medical device B is adapted for connecting at least two groups of electrically excitable cells A and B; wherein the connection between the at least two groups of electrically excitable cells A and B is bidirectional and comprises the following steps:
i. detecting the signals generated by the group A of electrically excitable cells with one of the two probes (1.1) of the medical device A (2);
ii. processing said signals and generating an output control signal with the signal processing means (1.4) of the medical device A (2);
iii. electrically stimulating the group A of electrically excitable cells or the group B of electrically excitable cells with the electrical pulse generator (1.3) and through the at least one electrode or electrode array (1.2) of the medical device A (2) respectively; wherein the at least one electrode or electrode array (1.2) is in contact with one or more cells of the group A or B of electrically excitable cells..

In a particular embodiment, the present invention is also directed to a cardiac pacemaker, defibrillator, neurostimulator, cochlear implant, glucose monitor or implantable infusion pump comprising the probe of the present invention in any of its embodiments, the monitoring device of the present invention in any of its particular embodiments, the medical device A or the medical device B of the present invention in any of their particular embodiments; preferably is implantable; more preferably is implantable *in vivo.*

In a particular embodiment, the medical devices A or B of the present invention are wireless. In a more particular embodiment, the medical devices A or B of the present invention are rechargeable. In a particular embodiment, the medical devices A or B of the present invention have a side length of between 0.5 and 10 cm.

In an embodiment, the medical device B is able to work bidirectionally.

### Methods for cell monitoring or stimulating

A further aspect is directed to a method for cell monitoring and stimulating comprising:
i. contacting one of the two probes (1.1) of the medical device A (2) as defined in the present invention with a group A of electrically excitable cells; wherein the electrically excitable cells generate measurable signals; and
ii. contacting the at least one electrode or electrode array (1.2) of the medical device A (2) with the group A of electrically excitable cells or with a group B of electrically excitable cells;
wherein the method is performed *in vitro* and/or *ex-vivo.*

In a particular embodiment, the method for cell monitoring or stimulating is performed *in vivo, in vitro* and/or *ex-vivo.*

In a further aspect, the invention is directed to a method for cell monitoring and stimulating comprising:
i. contacting one probe (1.1) of one of the at least two sets of probes (1.1) of the medical device B (3) as defined in the invention and one of the at least two electrodes or electrode arrays (1.2) of the medical device B (3) with the group A of electrically excitable cells;
ii. contacting other probe (1.1) of the at least two sets of probes (1.1) of the medical device B (3) as defined and other of the at least two electrodes or electrode arrays (1.2) of the medical device B (3) as defined with one group B of electrically excitable cells;
wherein the method is performed *in vitro* and/or *ex-vivo.*

In a particular embodiment, the method for cell monitoring or stimulating is performed *in vivo, in vitro* and/or *ex-vivo.*

### Uses

In a further aspect, the invention is directed to the use of the medical device A (2) of the invention for cell monitoring and stimulating *in vitro* and/or *ex-vivo;* preferably for electrically excitable cells.

In a particular embodiment, the use of the medical device A for cell stimulating is preferably for electrical cell stimulation and/or electrical cell neuromodulation; more preferably for electrical neural cell stimulation and/or for electrical neural cell neuromodulation; even more preferably for electrical cell growth stimulation and/or for electrical cell growth neuromodulation.

In a particular embodiment, the use of the medical device A (2) of the invention is for cell monitoring *in vitro* and/or *ex-vivo;* preferably for electrical cell monitoring *in vitro* and/or *ex-vivo;* more preferably for electrical neural cell monitoring *in vitro* and/or ex-*vivo.*

In a further aspect, the invention is directed to the use of the medical device B (3) of the invention for cell monitoring and stimulating *in vitro* and/or *ex-vivo.*
In a particular embodiment, the use of the medical device B (3) for cell stimulating is preferably for electrical cell stimulation and/or electrical cell neuromodulation; more preferably for electrical neural cell stimulation and/or for electrical neural cell neuromodulation; even more preferably for electrical cell growth stimulation and/or for electrical cell growth neuromodulation.

In a particular embodiment, the use of the medical device B (3) of the invention is for cell monitoring *in vitro* and/or *ex-vivo;* preferably for electrical cell monitoring *in vitro* and/or *ex-vivo;* more preferably for electrical neural cell monitoring *in vitro* and/or ex-*vivo.*

In the context of the invention, the term "neuromodulation" is directed to the electrical modulation of the neural network activity for directing the electrical signaling and/or favoring the axonal regeneration, synapse formation and/or synapse plasticity.
In a particular embodiment, the device of the present invention is in use as such or as part of another device in a cell culture media.

### Methods of treatment and/or prevention

In a further aspect, the invention is directed to a method of treatment and/or prevention of a disease, which method comprises implanting the medical device A (2) as defined in the present invention to a subject in need of such a treatment; wherein the disease comprises a malfunction or an interruption of signal transmission among different groups of electrically excitable cells.

In a further aspect, the invention is directed to a method of treatment and/or prevention of a disease, which method comprises implanting the medical device B (3) as defined in the present invention, to a subject in need of such a treatment; wherein the disease comprises a malfunction or an interruption of signal transmission among different groups of electrically excitable cells.

In a particular embodiment, implanting is surgically implanting; preferably *in vivo* surgically implanting.

As used herein, the term "treatment" or derivations thereof include the eradication, removal, reversion, alleviation, modification, or control of a disease.

The subject or the subject in need which is treated is a human or animal subject, preferably a human subject.

In an embodiment, the invention is also directed to the prevention of a disease, and in particular chronic pain, epilepsy, Parkinson's disease, sacral nerve pelvic disorders and incontinence, spinal cord traumatic disorders or injuries and spinal cord ischemic disorders or injuries.

The term "prevention" or derivations thereof refer to the avoiding or minimizing of the onset or development of the disease, in particular chronic pain, epilepsy, Parkinson's disease, sacral nerve pelvic disorders and incontinence, spinal cord traumatic disorders and spinal cord ischemic disorders.

### Electrical stimulation

The present invention is also directed to a method of treatment and/or prevention of chronic pain, epilepsy, Parkinson's disease, sacral nerve pelvic disorders and incontinence, spinal cord traumatic disorders and spinal cord ischemic disorders, which method comprises administering to a subject in need of such a treatment a therapeutically effective amount of electrical stimulation; wherein said electrical stimulation is administered using the medical device A of the present invention in any of its particular embodiments or the medical device B of the present invention in any of its particular embodiments.

Electrical stimulation relies on the ability of "electrically excitable cells" to generate an action potential in response to a depolarization of the membrane potential above an excitation threshold when an electrically excitable cell or group of cells is in contact with, or impaled upon, an electrode (e.g. during patch- or voltage- clamping), and a injection current or electrical pulse of a certain value (over a threshold) is applied. For example, a cardiomyocyte cell will require an injection current of about 1 nA to cause a membrane depolarization of about 10 mV, and thereby generate an action potential. In similar circumstances, other electrically active cell types may require injection currents of between 1-10 nA to cause depolarization. This in-situ electrical stimulation may allow the activation of voltage-dependent ionic channels present in the membrane of electrically excitable cells such as in neural cells or neurons. For example, it allows the control of key cellular messengers, such as calcium ions, that can lead to the stimulation of neural circuits in neurons. An expert in the art may determine the dosage and/or frequency of the injection current or electrical pulse of a certain value (over a threshold) applied which are effective according to the cells chosen.

In a particular embodiment of the present invention, the expression "administered" is related with the delivery of electrical stimulation; preferably of electrical signals, in particular of sinusoidal electrical signals or an electrical pulse train; more preferably to one or several neural cells of the subject in need.

In a particular embodiment, the electrical stimulation is electrical nerve stimulation.

In a particular embodiment, any of the methods of treatment and/or prevention of the present invention further comprises the following step:
i. applying a therapeutically effective amount of one or more electrical pulses, preferably to one or several electrically excitable cells of the subject in need; preferably to one or several neural cells of the subject in need.
   In a particular embodiment, any of the methods of treatment and/or prevention of the present invention further comprises the following steps:
   i. positioning the medical device A or B of the present invention in contact (i.e. electrical contact) with a selected portion of the subject in need of such a treatment, and
ii. applying a therapeutically effective amount of one or more electrical pulses.

In a particular embodiment, the subject in need of the present invention is a patient.

In a particular embodiment, any of the methods of treatment and/or prevention of the present invention are performed *in vivo.*

Non-limiting examples of portions of the subject in need of the treatment of the present invention are tissues of the subject comprising electrically excitable cells; preferably excitable cells selected from neuronal, cardiac, or muscle cells; preferably neural and/or cardiac cells; more preferably neural cells. For example, a portion of the subject in need might be selected from a portion of the brain, a portion of the spinal cord, nerves such as the sacral nerve, bundles of nerve fibers and nerve fibers; preferably a portion of the brain, the spinal cord or nerves.

In a particular embodiment, the electrical stimulation of the method or treatment of the present invention is therapeutically effective.

In a particular embodiment, the electrical stimulation of the method or treatment of the present invention is for continuous administration.

In another particular embodiment, the electrical stimulation of the method or treatment of the present invention is for administration once every three months, once every two months, once a month, once every three weeks, once every two weeks, once a week, or once or several times a day.

In a preferred embodiment of the present invention, the at least one electrode or electrode array of the medical devices are contacting the cell; preferably contacting the cell surface.

In a particular embodiment, any of the methods of treatment and/or prevention of the present invention further comprises the step of connecting the signal transmission among different groups of electrically excitable cells by the implantation of the medical device A or B.

### EXAMPLES

The invention is illustrated by means of the following examples that in no case limit the scope of the invention.

### Example 1 - Sensors for neural activity detection

A sensor was developed in the present experiment for neural activity detection. Said sensor comprises: commercial tunnel magneto resistance (TMR) sensors such as TMR-2922 or TMR-9003 (by MultiDimension), a printed circuit board specially developed for the experiment and a flat support. The sensors were coated with a texturized surface (a grating surface) of polydimethylsiloxane (PDMS) or polystyrene (PS) (see Example 2 below) and tested for the detection of neural activity produced by a spinal slice on bench. A photograph of the final sensor device used in the experiment is shown in Figure 2.

### Detectivity examples:

### First example

Two of the sensors (comprising a TMR-2922 sensor) were mounted in a differential configuration, i.e. placing one sensor closer to the magnetic field source of interest than the second sensor, which is at a further distance from the source, so that the first sensor only detects external noise signals (Figure 4 (a)). The detectivity output of both sensors was subtracted, collected by an amplifier (Figure 3, line (a)) and compared with the detectivity output of a single sensor (comprising a TMR-2922 sensor) (Figure 3, line (b)).

The inventors observed that the use of two sensors in a differential configuration allows the suppression of external magnetic signals. The on-bench magnetic field source produced a sinusoidal magnetic field of 40 µT at 128 Hz, said signal was the only signal present in the detectivity spectrum of the sensors in differential configuration (Figure 3, line (a)). However, as shown in Figure 3, the effect of the 50Hz and successive harmonics is evident in the detectivity of one individual sensor (Figure 3, line (b)), while it is absent in the corresponding curve of two sensors in differential configuration (Figure 3, line (a)). In said detection architecture, the overall intrinsic noise was Sqrt(2) higher than the independent signals of the sensors, as expected because their signals are uncorrelated. This increase in the detectivity is shown in Figure 3.

Figure 3 (line (a)) shows that the attenuation achieved for the unwanted external signals is remarkable for the sensors in differential configuration, while the detectivity remains mostly below the 1nT/Sqrt(Hz) threshold required for neural activity detection.

Therefore, when using two sensors in a differential configuration, the overall output will display only the signal of interest with a reduced external noise. Because, in addition, the saturation field is high (mT), the sensors allow recording a whole magnetic field (background + signal) and, at the same time, discriminating the signal of interest from the noise.

### Second example

In a second example, a significantly lower sinusoidal magnetic field of 3 nT at 16 Hz, was emitted by a magnetic source. Two sensors (sensor 1 and sensor 2) were used in a differential configuration to measure said signal as showed in Figure 4(a). Results showed that said signal was not resolved by the individual sensor 1(comprising a TMR-2922 sensor) in close proximity to the source (see Figure 4(b)). In addition, the second individual sensor 2 (comprising a TMR-2922 sensor) was detecting only external noise (see Figure 4(c)). In contrast, the output of both individual sensors mounted in a differential configuration showed a signal wherein the magnetic field of interest was resolved (Figure 4(d)).

### Example 2 -Sensors for neural activity detection

As stated in Example 1, the sensors used in the examples comprised a polymeric texturized surface (a grating surface) of polydimethylsiloxane (PDMS) or polystyrene (PS). The sensor coating process was as follows: first, a mold was fabricated by spin coating a positive photoresist (AZ9260) film over a Si wafer. The film thickness was adjusted according to the required height by adjusting the spin coating speed. As such, thicknesses ranging from 4.5 to 7 µm were obtained at spin speeds of 5000-3500 rpm. Optical lithography was then performed by using a maskless laser direct writing (Heidelberg DWL66). Then, a micropatterned polymeric layer coating sensor was obtained. A PDMS replica mold of this micropatterned polymeric layer was produced by casting the PDMS precursors (1:10 initiator) and using a curing protocol consisting of 24 hours at room temperature, 1 hour at 70°C and finally 30 minutes at 130°C after detaching. Figure 5 shows an example of the grating texturized surface with periods of ca. 20 µm (see Figure 5 top) produced on PS and PDMS polymeric layers.

### Example 3 - Organotypic spinal slices cultures over sensors

Organotypic spinal slices were cultured interfacing texturized polystyrene (PS) and polydimethylsiloxane (PDMS) coatings over sensors described in the previous examples.

Spinal cord slices, obtained from E12-E13 mouse embryos, were grown on grooved-PDMS and grooved-PS polymeric layers for 2 weeks and confocal microscopy was used to test the adhesion and growth of such cultures. Cultures interfaced to the two polymeric layers coating the sensors described above were also compared to control ones grown on standard glass coverslips. As showed on Figure 6 left column, the neuron-specific microtubules (β-tubulin III) and the neurofilament H (Smi-32) were imaged using immunofluorescence techniques. Nuclei are visualized by DAPI. Fluorescence Ca²⁺ imaging was used for synapse formation and activity after *in vitro* growth. Clusters of neurons stained with the membrane-permeable Ca²⁺ dye Fluo-4-AM (Figure 6 second left column) were simultaneously visualized and monitored. Recordings were performed at the premotor region in the ventral zone were spontaneous activity is more prominent. The cell ability to generate repetitive Ca²⁺ oscillations was measured and compared in the two different polymeric layers, grooved PS (Figure 6c) and grooved PDMS (Figure 6b), and compared to glass coverslips (Figure 6a, control). In Figure 6 (right panel, middle tracings), traces represent spontaneously fluorescence recordings from active neurons, fully blocked by tetrodotoxin (a blocker of voltage-gated, fast Na⁺ channels) application (Figure 6, right panel, right tracings).

The motor and sensory axons outgrowing from the ventral spinal cord and the dorsal root ganglia, respectively, were characterized by performing double immunostaining for the anti-β-tubulin III antibody together with the primary mouse anti-neurofilament H (Smi-32) antibody (marker for projecting neurons, DRG neurons and motoneurons).

Figure 6 shows immunofluorescence images (left column, scale bar 500 µm) of neuron-specific microtubules and neurofilament H of spinal cord explants *in vitro,* interfaced to (a) glass coverslips (control) and to polymeric layers: (b) grooved PDMS polymeric layer and (c) grooved PS polymeric layer. Higher magnification fluorescence images of clusters of neurons stained with membrane-permeable Ca²⁺ dye Fluo-4 AM are shown in the second column (scale bar 50 µm). In addition, Figure 6 shows recordings of representative traces for spontaneous synaptic activity measured as live calcium fluorescence imaging as fractional amplitude increase (ΔF/F₀) in the absence (third column) and presence of tetrodotoxin (fourth column).

In addition, Figure 7 shows, in spinal slices interfaced to (a) glass coverslips (control) in the absence of structured interfaces, random axons regrowth with no preferred orientation, usually organized centrifugally around the spinal tissue (Figure 7 A, left panel, scale bar 500 µm). In contrast, when the spinal cord slices were interfaced to patterned polymeric layers such as grooved PDMS and grooved PS coatings, an orientation of the regenerative axons was evident (Figure 7A, middle (b) and right (c) panels, scale bar 500 µm). This can be further appreciated in the confocal magnification images showed on Figure 7, panel B and C.

Thus, texturized polystyrene (PS) and polydimethylsiloxane (PDMS) sensor coatings described in example 2 were biocompatible, maximized the cell/coating interaction, induced neuronal fibers alignment and facilitated the formation of neurite bundles with well-defined directionality, similar to those found along the spinal cord.

### Example 4 - Neural activity of organotypic spinal slices over sensors

The neural activity of organotypic spinal slices cultured interfacing texturized polystyrene (PS) and polydimethylsiloxane (PDMS) coated sensors described in the previous examples was tested. Extracellular field potential recording was performed on slices at 14-21 DIV, using low resistance glass micropipettes filled with the same extracellular saline solution. Neural activity recordings were achieved in the vicinity of the axon bundles. Spontaneous activity was of variable frequency and intensity depending on the position and the size of the investigated bundle of axons. To have more reproducibility in the measurements, the neural network was pharmacologically synchronized by disinhibiting it through Bicuculline 25 µM/Strychnine 1 µM application (GABAA receptor and Glycine receptor antagonists, respectively). This resulted in a rhythmic and periodic activity of greater intensity and duration if compared to signals found during spontaneous activity. Therefore, this protocol was considered as the best condition for sensing low magnetic fields at the axon bundles level. The axonal activity was monitored after 14-21 DIV, by fluorescence Ca²⁺ imaging. Figure 8 left shows snapshots of axons outgrowing the spinal cord stained with membrane-permeable Ca²⁺ dye Fluo-4-AM (scale bar 100 µm)grown on (a) control glass, (b) grooved PDMS polymeric layer and (c) grooved PS polymeric layer. In addition, recordings of representative traces for axonal activity measured as live calcium fluorescence imaging recorded as fractional amplitude increase (ΔF/F₀) in the absence (second column) and in the presence of Bicuculline/Strychnine (third column) are shown.

Thus, in the present experiment, it was observed that organotypic spinal slices were active when cultured interfacing the texturized polystyrene (PS) and polydimethylsiloxane (PDMS) coatings of the sensors.

### Example 5 - Real-time detection with sensors of the neural activity of organotypic spinal slices

The neural activity of organotypic spinal slices was measured by using the PDMS coated sensors described in the previous examples. During the experiment, the neural activity was measured at the same time by live calcium imaging of the ventral premotor circuit.

Figure 9 (a) shows a schematic of the setup of the experiment wherein two magnetic sensors were used in a differential configuration (the two magnetic sensors were placed at different distances of the spinal slice). The neural network of a spinal cord organotypic slice was interfacing the texturized surface of the sensor of the top (Figure 9 (b)).

The neural network was pharmacologically synchronized by disinhibiting it through Bicuculline 25 µM/Strychnine 1 µM application (GABAA receptor and Glycine receptor antagonists, respectively). This condition is of great importance to synchronize signals and enhance the probability to read the occurrence of the pulsed field variation that takes place during the action potential displacement along the axons. Figure 10 shows the results of the measurements performed simultaneously by Ca²⁺ imaging and by magnetic sensing on the neural network of a spinal cord organotypic slice. Figure 10 (A) (left side) shows a field image of the ventral horn of the spinal cord organotypic slice which was loaded with Fluo-4-AM (scale bar 50 µm). In addition, synaptic activity was measured as live calcium fluorescence (imaging fractional amplitude increase (ΔF/F0) over time) in the presence of Bicuculline/Strychnine (Figure 10A, right side). Figure 10 (B) shows the simultaneous magnetic detection results obtained during the calcium fluorescence imaging experiment described above. In particular, it shows the representative traces for spontaneous synaptic activity recorded as voltage (µV) over time (ms) in the presence of (i) Bicuculline/Strychnine and (ii) Tetrodoxin.

Results confirmed that the sensors of the present invention were able to detect neural activity of a culture. The recorded traces (voltage (µV) over time (ms)) showed clear peaks (Figure 10 (B), traces (i)) and the detected magnetic field is in the order of tenths of nT. Importantly, the signal displayed on Figure 10 has not been averaged nor post-treated in any way. Moreover, the neuronal nature of the recorded signal was removed upon tetrodotoxin administration, which impairs action potential spreading along the axons (Figure 10 (B), traces (ii)), the output of the magnetic sensors became silent in these conditions.

The peak-to-peak inter-event-interval (IEI) occurrence of spontaneous Ca²⁺ episodes in the cultured cells during Bicuculline/Strychnine-induced activity was quantified (in seconds). IEI values were independently obtained using the magnetic sensors described above and calcium fluorescence. Figure 10 (C) shows the IEI data distributions obtained using both measuring methods. It was observed that the IEI data distribution dispersion obtained from the magnetic readings is significantly larger that the IEI data distribution dispersion obtained from the calcium imaging. However, the average IEIs data distribution dispersion obtained from the magnetic readings is only about double than that of the calcium imaging. This is a good ratio taking into account that presumably only the closest layers of the organotypic spinal slice are contributing to the signal detected by the sensors.

### Example 6 -Metallic electrodes with nanostructured surface

Flexible metallic nanostructured electrodes were obtained.

In particular, nanostructured electrodes comprising conducting nanowires placed vertically on a conducting flexible metallic sheet were developed by a route based in a template-assisted electrodeposition technique for the fabrication of nanowires (Figure 11 A). First, a nanoporous template was metalized on one of its sides with Au using sputtering or Joule evaporation. Then, the metalized Au layer was thickened using pulse-plating electrodeposition and the nanowires were grown inside the template by electrochemical deposition. After releasing the template, the metallic nanostructured electrode was ready. In addition, core-shell structures and multicore structures were fabricated by adding a step of pulsing plating electrodeposition. Au electrodes were obtained using a polycarbonate (PC) nanoporous membrane as template. The membrane, supplied by Whatman, had 100 nm pore diameter and 6 µm-thickness. In addition, the pore density of the template was 108 cm⁻². Metallic Au nanowires were grown filling the pores of the membrane by potentiostatic deposition using Orosene (ORE+4) electrolyte with a growth potential of -1.5 V (growth rate of 11 nm/s). To release the nanowires, the PC template was dissolved using dichloromethane followed by extensive washing in acetone, ethanol and deionized water. Ni electrodes were obtained following a similar method. Results showed that the obtained electrodes were flexible. Figure 11 shows (A) a sketch of the electrodes fabrication and (B) scanning electron microscopy (SEM) micrographs of the electrodes obtained. Figure 11 (B) inset illustrates the flexibility of the final electrodes. In addition, flexible nanostructured electrodes composed of vertical arrays of nickel (Ni-NWs) nanowires (NWs) grown on top of Au substrates were obtained in a similar way as described above but using a Watts-type electrolyte composed by NiSO₄ (0.8 M), NiCl₂ (0.2 M) and H₃BO₃ (0.4 M), working at 45 °C, with a growth potential of -1.0 V vs. RE. Ni metal nanowires showed robustness and good mechanical properties. The impedance of the electrodes was measured at room temperature by a three-electrode cell using a Metrohm Autolab PGSTAT204 potentiostat with a Pt mesh as a counter electrode and an Ag/AgCI (3 M NaCI) electrode as reference. Measurements were carried out in phosphate buffer saline (PBS) with bovine serum albumin (0.2 mg mL-1), with a modulation of 10 mV at 250 Hz, in circular electrodes of 4 mm in diameter. Interestingly, we found the impedance of both Au-NWs and Ni-NWs electrodes to be very similar, 106 ± 3 Ω and 108 ± 3 Ω, respectively, which is less than half of the impedance measured in a planar Au electrode of the same area (232 ± 3 Ω), showing that nanostructure clearly reduced the impedance of the electrode as expected, due to a larger electrode effective area. Also, impedance values a 10% lower were observed when bending the nanostructured electrodes as described above. A denser nanostructured network covering more tightly the base surface would reduce further the final electrode impedance. We are presently working on obtaining this type of nanostructure.

### Example 7 - Biocompatibility of metallic nanostructured electrodes using embryonic neural progenitor cells

The biocompatibility of the metallic nanostructured electrodes described in Example 6 was studied *in vitro* using embryonic neural progenitor cells.

Prior to cell culture, the electrodes were sterilized by UV radiation in a biosafety cabinet and then functionalized by adsorbing low molecular weight poly-L-lysine molecules (PLL; 30,000 - 70,000 Da; 45 µg ml⁻¹). The homogeneity of the coating was confirmed by immunofluorescence by using PLL-FITC. Control glass substrates were functionalized following the same protocol than metallic electrodes. Embryonic neural progenitor cells (ENPCs) were obtained from cerebral cortices of E18 Wistar rat embryos. Adult female Wistar rats were provided by a commercial supplier (Harlan Ibérica, Spain) and sacrificed when gestation reached 16-18 days. A total of 5 independent cell cultures from 5 different animals with a minimum of 3 replicates per condition in each culture were carried out (≥ 15 arrays per condition). The viability of the so-isolated cells was 90 ± 4 % in all cases. Morphological evaluation of ENPCs cultures was performed at high- and low-density seeding conditions. For high-density assays, a total of 7.5·10⁴ cells contained in a small fraction of media (typically 20-50 µl) was seeded on the top part of each array and allowed to attach for 10 min. Immediately after, samples were completely covered with 500 µl of complete Neurobasal^{™} media containing: B-27 supplement (2 %), streptomycin (100 UI ml⁻¹), penicillin (100 UI ml⁻¹), and L-glutamine (1 mM). For low-density assays, cells were seeded at a density of 2.5·10⁴ cells cm⁻². After 2 h of adhesion in a sterile incubator at 37 °C in a CO₂ atmosphere (5 %), culture media were replaced and cultures maintained for up to 2 weeks. Culture media were half replaced every 3-4 days. Cell culture was monitored in the periphery of the substrates and control samples by using an Axiovert CFL-40 optical microscope with a coupled Axiocam ICC-1 digital camera (Zeiss). Results showed that electrodes were biocompatible with ENPCs. In particular, after two weeks, ENPCs properly attached and spread on top of PLL-coated Au-NWs platforms colonizing the totality of the substrate surface as shown in the scanning electron microscopy micrographs of Figure 13 (results on glass substrates were also obtained as control, see Figure 12). Detailed observation by field-emission scanning electron microscopy (FESEM) revealed a close contact of both somata and neurites with the NWs (Figure 13 (B)), without evidences of either neural cell membrane piercing or perforation. The viability degree of the cultures was then evaluated. In both the glass control and Au-NWs electrodes, a majority of the surface was covered by live cells. When quantified (Figure 14 (a)), cells cultured on nanostructured electrodes were similarly viable to those on control glass substrates (98.8 % with respect to control, p > 0.999). Differentiation to either neuronal or non-neuronal phenotypes was also investigated by immunofluorescence studies of map-2 (neuronal cytoskeleton protein) and vimentin (non-neuronal cytoskeleton protein) (Figure 14 (b)). In the nanostructured electrodes, there was a predominant presence of neuron-differentiated cells, as occurred on control substrates and expected for this cell type and these culture conditions. When quantified, cells on Au electrodes displayed a similar differentiation profile than that found on glass coverslips (map-2: p = 0.248; vimentin: p = 0.886).

### Example 8 - Biocompatibility of metallic nanostructured electrodes using primary hippocampal cultures

The biocompatibility of the metallic nanostructured electrodes was studied using primary hippocampal cultures. In particular, the Au nanostructured electrodes (Au-NWs), Ni nanostructured electrodes (Ni-NWs) described in Example 6 were studied. In addition, flat Au and Ni sheets (Au-Flat and Ni-Flat) were tested.

Dissociated hippocampal neurons were isolated from post-natal 2-3 days old Wistar rats. Au-F and Au-NWs electrodes were sterilized with three 5 minutes-long washes on milliQ H₂O previously filtered with a 0.22 µm cutoff filter (Merck Millipore). Samples were dried at 60 °C and their surfaces were activated under low-pressure air plasma (Harrick PDC-32G Plasma Cleaner) for 7 minutes at room temperature (RT), with RF coil powers of 9 Watt. A 20 minutes-long exposure to ultraviolet (UV)-radiation was finally used to sterilize the substrates. About 800 cells/mm² were plated onto Au-F and Au-NWs incubated at 37 °C, 5 % CO₂ in Neurobasal medium (Invitrogen) added with B-27 supplement (Thermo Fisher) and GlutaMAX (Thermo Fisher) both to a 1× final concentration. Gentamicin (Thermo Fisher) was also added to a final concentration of 5 µg/ml to prevent contaminations. Cultured cells were grown until 8-12 days *in vitro* (DIV) by renewing half of the medium once in this period.

Results showed that electrodes were biocompatible with primary hippocampal cultures.

### Example 9 - Electrical stimulation of hippocampal cultures using flexible metallic nanostructured electrodes

Au nanostructured (Au-NWs) electrodes as described in Example 6 and flat Au sheets (Au-Flat) were used to electrically stimulate hippocampal cultures.

For the electrical stimulation, the bottom of a 35 mm petri dish (Falcon^{®}) was modified to include either Au-Flat or Au-NWs squared sheets of about 6 × 6 mm. The junction between the petri and the Au-Flat or Au-NWs electrodes was sealed by PDMS silicon elastomer (Sylgard^{®} 184 - Down Corning Co.) This setting allowed positioning an electrical contact (a platinum wire) in direct contact with the Au-Flat and Au-NWs electrodes in a dry environment. Hippocampal cultures were grown for 10 DIV in the modified petri dishes. To deliver the electrical stimuli, the positive output of an STG 4002 stimulator (Multi Channel Systems) was plugged to the electrical contact (platinum wire) which contacted the Au-Flat and Au-NWs electrodes, as described above. The ground output was plugged with an Ag/AgCI pellet electrode submerged in the extracellular saline solution (Figure 15 (a)). Biphasic voltage steps from 0 to 1 V in steps of 200 mV were sent at 0.2 Hz and each phase lasted 300 µs for a total stimulus length of 25 s (Figure 15 (b)). The extracellular stimulus pattern was designed with MC_Stimulus II (Multi Channel Systems) and the stimulator was controlled by a computer. At the same time, calcium live imaging was recorded. Cells were loaded with the cell permeable Ca²⁺ dye Oregon Green 488 BAPTA-1 AM (Molecular Probes); 10 µL DMSO (Sigma-Aldrich) were added to the stock 50 µg of the dye and cultures were incubated with a final concentration of 4 µM for 30 min at 37 °C, 5 % CO₂. After incubation, the petri dish with the substrate was mounted in a fixed-stage upright microscope (Eclipse FN1, Nikon) and was continuously superfused at 5 mL/min rate, at RT, with extracellular saline solution of the following composition (mM): 150 NaCl, 4 KCl, 2 CaCl₂, 1 MgCl₂, 10 HEPES and 10 glucose (pH adjusted to 7.4 with NaOH; osmolarity 300 mOsm). The Ca²⁺-dye was excited at 488 nm with a mercury lamp; a dichroic mirror and ND filter (1/32) was used to separate the excitation light from the light emitted by the sample. Spontaneous calcium transients were recorded with a 20 × water immersion objective (UMPlanFI, 0.5 NA, Olympus) using an EMCCD camera (IXon Ultra 897, AndorTM, Oxford Instruments) controlled by a computer through NIS-elements D (Nikon). Images were acquired each 150 ms at 10 MHz readout compensating the read noise with ×250 electron multiplying (EM) gain.

First, 15 minutes of basal spontaneous activity were recorded in order to corroborate the state of health of tested cells in both conditions. Figure 15 (c) shows two representative snapshots of the imaged field of hippocampal dissociated cultures. A circular ROI (region of interest, circle) was drawn around spiking neurons, the mean grey value of the pixels in the ROIs was used to plot the calcium transient courses over time, as those on the right side of Figure 15 (c). Next, antagonists were applied (Sigma-Aldrich): APV 25 µM (glutamate NMDA receptor selective antagonist), bicuculline 10 µM (GABAA receptor antagonist) and CNQX 10 µM (glutamate AMPA/kainate receptor antagonist) to remove all ongoing synaptic activity and observe only stimulus-evoked activity. Evoked Ca²⁺ transients due to direct excitation of cells and generation of action potentials via Au-Flat and Au-NWs sheets electrical stimulation were detected under these experimental conditions (Figure 15 (d), upper panel). The neuronal nature of the recorded calcium episodes was ensured by applying 1 µM TTX (a voltage-gated, fast Na⁺ channel blocker; Latoxan) (Figure 15 (d), lower panel), which totally abolished the neuronal cells ability to fire action potentials. All offline analysis were accomplished by the ImageJ software (NIH) and the Clampfit software (pClamp suite, 10.4 version; Axon Instruments). Similar tests were performed with Ni nanostructured electrodes.

Au and Ni metallic nanostructured electrodes results showed that Ni electrodes increase neural cell survival, boost neuronal differentiation and reduce glial cell content with respect to their flat counterparts. In addition, Au and Ni nanostructured electrodes induced a significant reduction in glial cell density on primary hippocampal cell cultures when compared to flat substrates. Under electrical stimulation, the nanostructured electrodes sustain intracellular calcium dynamics compatible with functional neural networks.

## Claims

1. A probe (1.1) comprising at least one sensor or sensor array adapted for monitoring a signal; wherein the probe (1.1) is biocompatible and optionally implantable; wherein the signal monitored by the at least one sensor or sensor array is a magnetic signal.

2. The probe (1.1) according to claim 1; wherein the at least one sensor or sensor array adapted for monitoring a signal comprises two surfaces;
wherein a polymeric layer is coating at least one surface of the at least one sensor or sensor array.

3. The probe (1.1) according to any of claims 1 or 2; wherein the sensor is a magneto resistance sensor and/or wherein the magnetic signal is generated by one electrically excitable cell or by a group of electrically excitable cells.

4. The probe (1.1) according to any of claims 2 to 3; wherein the polymeric layer comprises:
(a) a textured surface; and
(b) a bulk portion; and
wherein the textured surface of the polymeric layer comprises a micropatterned structure; and/or
wherein the textured surface comprises a plurality of protrusions with a grating shape with a height of between 1 µm and 10 µm, a width of between 1 µm and 20 µm, and an interval between the protrusions of between 1 µm and 30 µm; and
wherein the polymer of the polymer layer is selected from poly(methyl methacrylate) (PMMA), polycarbonate (PC), polystyrene (PS), polydimethylsiloxane (PDMS), polypropylene (PP), polyethylene (PE), polyglycolide (PGA), poly(propylenefumarate) (PPF), polycyanoacrylate (PCA), polycaprolactone (PCL), poly(glycerol sebacate) (PGS), poly(glycerol sebacate acrylate) (PGSA), polyvinylidenefuoride (PVDF), polyvinylidene chloride (PVDC), polyethylene terephthalate (PET), polybutylene therephtalate (PBT), polyphenylene oxide (PPO), polyvinyl chloride (PVC), cellulose acetate (CA), cyclic olefin copolymer (COC), ethylene vinyl acetate (EVA), ethylene vinyl alcohol, (EVOH), polytetrafluoroethylene (PTFE), fluorinated ethylene propylene (FEP), perfluoroalkoxy (PEA), ethylene tetrafluoroethylene (ETFE), polyethersulfone (PES), chlorinated poly ethylene (CPE), polylactic acid (PLA), poly 3-hydroxybutyrate (P3HB), polybutylene adipate (PBA), polyethylene adipate (PEA), polybutylene succinate (PBS), polyphenylene sulfide (PPS), polysulfone (PSU), polytrimethylene terephthalate (PTT), polyurethane (PU), polyvinyl acetate (PVA), polyvinylidene chloride (PVDC), styrene acrylonitrile (SAN), polyetherketones (PEEK), polyvinylalcohol (PVA), polyhydroxyethylmethacrylate (PHEMA), poly(N-isopropylacrylamide) (PNIPAAm) and mixtures thereof; and/or
wherein the at least one sensor or sensor array is a spintronic sensor or spintronic sensor array; and wherein the at least one sensor or sensor array has detectivity values below 10 nT/Sqrt(Hz) at frequencies below 100 Hz; and wherein the at least one sensor or sensor array is non-cooled.

5. A monitoring device (1) comprising
i. two probes (1.1) as defined in any of claims 1 to 4; wherein the two probes (1.1) are physically separated a distance of between 0.1 and 100 mm; and
ii. signal processing means (1.4) adapted for processing the signals of the probes;
wherein the two probes (1.1), and the signal processing means (1.4) are operatively connected; and
wherein the monitoring device (1) is optionally implantable.

6. A method for electrically excitable cell monitoring comprising the steps of:
i. stablishing a connection between the monitoring device (1) as defined in claim 5 with one or more electrically excitable cells; and
ii. monitoring the measureable signals with the monitoring device as defined in claim 5;
wherein the method is performed *in vitro* and/or *ex-vivo.*

7. Use of the monitoring device according to claim 5 for cell monitoring; preferably *in vitro* and/or *ex-vivo.*

8. A medical device A (2) comprising:
i. two probes (1.1) as defined in any of claims 1 to 4; wherein the two probes (1.1) are physically separated a distance of between 0.1 and 100 mm;
ii. at least one electrode or electrode array (1.2) adapted for applying an electrical pulse of a certain value; and
iii. an electrical pulse generator (1.3) adapted for generating electrical pulses of a certain value; wherein the at least one electrode or electrode array (1.2) and the electrical pulse generator (1.3) are operatively connected; and
iv. signal processing means (1.4) adapted for processing the signals of the at least one probe (1.1) and for generating an output control signal for the electrical pulse generator (1.3); wherein the two probes (1.1) are operatively connected with the signal processing means (1.4);
wherein the electrical pulse generator (1.3) and the signal processing means (1.4) are operatively connected;
wherein the at least one probe (1.1), the at least one electrode or electrode array (1.2), the electrical pulse generator (1.3) and the signal processing means (1.4) are operatively connected; and
wherein the medical device A (2) is optionally implantable.

9. A method for cell monitoring and stimulating comprising
i. contacting one of the two probes (1.1) of the medical device A (2) as defined in claim 8 with a group A of electrically excitable cells; wherein the electrically excitable cells generate measurable signals;
ii. contacting the at least one electrode or electrode array (1.2) of the medical device A (2) as defined in claim 8 with the group A of electrically excitable cells or with a group B of electrically excitable cells;
wherein the method is performed *in vitro* and/or *ex-vivo.*

10. A medical device B (3) comprising:
i. at least two sets of probes; wherein each set of probes comprises two probes (1.1) as defined in any of claims 1 to 4; wherein in each of the set of probes, the probes (1.1) are physically separated a distance of between 0.1 and 100 mm;
ii. at least two electrodes or electrode arrays (1.2) adapted for applying an electrical pulse of a certain value;
iii. an electrical pulse generator (1.3) adapted for generating electrical pulses of a certain value; wherein the at least one electrode or electrode array (1.2) and the electrical pulse generator (1.3) are operatively connected; and
iv. signal processing means (1.4) adapted for processing the signals of the at least two sets of probes (1.1) and for generating an output control signal for the electrical stimulator (1.3); wherein the two probes (1.1) are operatively connected with the signal processing means (1.4);
wherein the electrical pulse generator (1.3) and the signal processing means (1.4) are operatively connected;
wherein the at least two sets of probes (1.1), the at least two electrodes or electrode arrays (1.2), the electrical pulse generator (1.3) and the signal processing means (1.4) are operatively connected so that the medical device B (3) is able to work bidirectionally;
wherein the medical device B (3) is optionally implantable.

11. The medical device A(2) according to claim 8 and/or the medical device B(3) according to claim 10, wherein the at least two electrodes or electrode arrays (1.2) adapted for applying an electrical pulse of a certain value comprise:
a) a textured surface with a plurality of nanowires, and
b) a bulk portion, wherein the bulk portion is a sheet, preferably a metallic sheet.

12. The medical device A(2) according to claim 11 and/or the medical device B(3) according to claim 11, wherein the plurality of nanowires of the at least two electrodes or electrode arrays are a plurality of metallic nanowires; wherein the metal of the metallic nanowires is selected from Au, Pt, Co, Fe, Ni and combinations thereof; and wherein the bulk portion is a metallic sheet where the metal of the metallic sheet is selected from Au, Pt, Co, Fe, Ni and/or combinations thereof; and wherein the metallic sheet has a thickness of below 1.5 microns; and wherein the plurality of nanowires have a diameter of between 1 and 500 nm; and/or
wherein at least two electrodes or electrode arrays are obtainable by growing nanowires on a metal sheet using a template-assisted electrodeposition method.

13. A method for cell monitoring and stimulating comprising:
i. contacting one probe (1.1) of one of the at least two sets of probes (1.1) of the medical device B (3) as defined in claim 10 or in claims 11 or 12; and one of the at least two electrodes or electrode arrays (1.2) of the medical device B (3) with a group A of electrically excitable cells;
ii. contacting other probe (1.1) of the at least two sets of probes (1.1) of the medical device B (3) and the other of the at least two electrodes or electrode arrays (1.2) of the medical device B (3) with one group B of electrically excitable cells;
wherein the method is performed *in vitro* and/or *ex-vivo.*

14. Use of the medical device A (2) according to any of claims 8 or 11-12 for cell monitoring and stimulating *in vitro* and/or *ex-vivo.*

15. Use of the medical device B (3) according to any of claims 10 or 11-12 for cell monitoring and stimulating *in vitro* and/or *ex-vivo.*
